# EUROPÄISCHE PATENTANMELDUNG

(11) **EP 4 163 928 A1**
(43) Veröffentlichungstag der Anmeldung: **12.04.2023**
(21) Anmeldenummer: 22200072.1
(22) Anmeldetag: 06.10.2022
(51) Int. Cl.: G16H 40/20, G16H 10/20, G16H 10/60, G16H 80/00

(54) **PATIENTENMANAGEMENTSYSTEM UND VERFAHREN ZUM PATIENTENMANAGEMENT**

(30) Priorität: 08.10.2021 DE 102021211393
(71) Anmelder: Praevida GmbH, 69123 Heidelberg (DE)
(72) Erfinder: Baumgart, André, CH-6234 Triengen (CH); Kramer, Birgit, 69221 Dossenheim (DE); Fischer, Joachim, 69115 Heidelberg (DE)
(74) Vertreter: Ullrich & Naumann PartG mbB

(57) **Zusammenfassung**

Ein Patientenmanagementsystem, insbesondere zur Unterstützung des Arztpraxisbetriebs, vorzugsweise im Bereich der Allgemeinmedizin und/oder der hausärztlichen Versorgung, umfassend: ein Patienteninteraktionsmodul zur Bereitstellung eines geschützten digitalen Patientenbereichs, wobei das Patienteninteraktionsmodul dazu ausgebildet ist, einem Patienten in Reaktion auf eine Registrierungsanfrage ein persönliches Benutzerkonto zuzuordnen und im Rahmen des persönlichen Benutzerkontos patientenspezifische Daten zu erfassen, und ein Priorisierungsmodul zur Bestimmung der Dringlichkeit eines Patientenanliegens, wobei das Priorisierungsmodul dazu ausgebildet ist, eine Dringlichkeitsentscheidung auf Basis der erfassten patientenspezifischen Daten zu ermitteln, so dass in Abhängigkeit der Dringlichkeitsentscheidung eine Weiterleitung des Patienten erfolgt. Des Weiteren ist ein entsprechendes Verfahren zum Patientenmanagement offenbart.

## Beschreibung

Die Erfindung betrifft ein Patientenmanagementsystem, insbesondere zur Unterstützung des Arztpraxisbetriebs, vorzugsweise im Bereich der Allgemeinmedizin und/oder der hausärztlichen Versorgung.

Des Weiteren betrifft die Erfindung ein Verfahren zum Patientenmanagement, insbesondere zur Unterstützung des Arztpraxisbetriebs.

Derzeit gibt es in Deutschland rund 54.000 Praxen mit hausärztlicher Versorgung. Dabei basiert das bisherige System im Wesentlichen auf der Idee der Einzelarztpraxis und hat bislang weitgehend die Möglichkeiten der Digitalisierung ignoriert. Zwar haben IT-gestützte Praxisinformationssysteme Einzug in die Behandlung erhalten, sie dienen jedoch weitgehend der Dokumentation und Abrechnung innerhalb eines durch stark reglementierte Vergütungsstrukturen charakterisierten Systems. Wirtschaftlich erfolgreich kann eine als Einzelarztpraxis geführte Hausarztpraxis nur dann agieren, wenn Ärzte bereit sind, bis zu 50 oder 60 Patienten pro Tag zu sehen und diese in einem eng getakteten Rhythmus abzuarbeiten. In einer derartigen Sprechstunde bleibt weder Vergütung noch Zeit für eine (wirksame) Gesundheitsförderung des Patienten. Eine umfassende Erhebung der Bedürfnisse der Patienten, ihrer Ressourcen, ihrer sonstigen Lebensumstände ist praktisch ausgeschlossen, da dies durch die Vergütungssysteme nicht abgebildet ist.

Ebenso findet keine Nachbefragung statt, kaum ein Arzt weiß wirklich, wie es seinen Patienten nach einer medizinischen Verordnung geht. Ein konkretes Beispiel dafür sind die (insbesondere in Deutschland im internationalen Vergleich gesehen) stark verbesserungsfähigen Behandlungsergebnisse beim Bluthochdruck, einem der wichtigsten Risikofaktoren für die häufigste Todesursache, nämlich Herz-Kreislauferkrankungen. Von 100 Personen mit Bluthochdruck wissen nur etwa zwei Drittel davon und nur bestenfalls ein Drittel ist adäquat behandelt.

Der vorliegenden Erfindung liegt daher die Aufgabe zugrunde, ein Patientenmanagementsystem und ein Verfahren zum Patientenmanagement der eingangs genannten Art derart auszugestalten und weiterzubilden, dass eine möglichst effiziente und wirksame Abwicklung des Patienten ermöglicht ist.

Erfindungsgemäß wird die voranstehende Aufgabe durch die Merkmale des Anspruchs 1 gelöst. Danach ist ein Patientenmanagementsystem angegeben, insbesondere zur Unterstützung des Arztpraxisbetriebs, vorzugsweise im Bereich der Allgemeinmedizin und/oder der hausärztlichen Versorgung, wobei das in Rede stehende System umfasst:
ein Patienteninteraktionsmodul zur Bereitstellung eines geschützten digitalen Patientenbereichs, wobei das Patienteninteraktionsmodul dazu ausgebildet ist, einem Patienten in Reaktion auf eine Registrierungsanfrage ein persönliches Benutzerkonto zuzuordnen und im Rahmen des persönlichen Benutzerkontos patientenspezifische Daten zu erfassen,
ein Priorisierungsmodul zur Bestimmung der Dringlichkeit eines Patientenanliegens, wobei das Priorisierungsmodul dazu ausgebildet ist, eine Dringlichkeitsentscheidung auf Basis der erfassten patientenspezifischen Daten zu ermitteln, so dass in Abhängigkeit der Dringlichkeitsentscheidung eine Weiterleitung des Patienten erfolgt.

Die voranstehende Aufgabe ist des Weiteren durch die Merkmale des Anspruchs 13 gelöst. Danach ist ein Verfahren zum Patientenmanagement angegeben, insbesondere zur Unterstützung des Arztpraxisbetriebs, wobei mit einem Patienteninteraktionsmodul einem Patienten ein geschützter digitaler Patientenbereich bereitgestellt wird, wobei das Patienteninteraktionsmodul dem Patienten in Reaktion auf eine Registrierungsanfrage ein persönliches Benutzerkonto zuordnet, wobei durch das Patienteninteraktionsmodul im Rahmen des persönlichen Benutzerkontos patientenspezifische Daten erfasst werden, wobei mittels einem Priorisierungsmodul die Dringlichkeit eines Patientenanliegens bestimmt wird, wobei das Priorisierungsmodul eine Dringlichkeitsentscheidung auf Basis der erfassten patientenspezifischen Daten ermittelt, und wobei in Abhängigkeit der Dringlichkeitsentscheidung eine Weiterleitung des Patienten im Patientenmanagementsystem erfolgt.

In erfindungsgemäßer Weise ist zunächst erkannt worden, dass es von erheblichem Vorteil ist, wenn bereits im Vorfeld einer ärztlichen bzw. medizinischen Konsultation und/oder Behandlung patientenspezifische Daten erfasst werden. So kann eine enorme Zeitersparnis und Effizienzsteigerung erreicht werden, da der Praxisbetrieb von aufwändigen, manuellen Datenerfassungen, insbesondere vor Ort in der Praxis, weitestgehend befreit werden kann. Erfindungsgemäß ist dazu ein Patientenmanagementsystem zur Unterstützung des Arztpraxisbetriebs vorgesehen, das ein Patienteninteraktionsmodul zur Bereitstellung eines geschützten digitalen Patientenbereichs aufweist. Das Patienteninteraktionsmodul ist dazu ausgebildet, einem Patienten in Reaktion auf eine Registrierungsanfrage ein persönliches Benutzerkonto zuzuordnen. Im Rahmen des persönlichen Benutzerkontos werden patientenspezifische Daten erfasst, nämlich bereits im Vorfelde einer ärztlichen bzw. medizinischen Konsultation. Somit können im Vorfeld der ärztlichen bzw. medizinischen Konsultation beispielsweise Beschwerden und/oder andere (wesentliche) Patientenanliegen computergestützt erfasst werden. Des Weiteren ist erfindungsgemäß ein Priorisierungsmodul vorgesehen, das zur Bestimmung der Dringlichkeit eines Patientenanliegens dient. Im Konkreten ist das Priorisierungsmodul dazu ausgebildet, eine Dringlichkeitsentscheidung auf Basis der erfassten patientenspezifischen Daten zu ermitteln, so dass in Abhängigkeit der Dringlichkeitsentscheidung eine Weiterleitung des Patienten im Patientenmanagementsystem erfolgt.

Folglich ist mit dem erfindungsgemäßen Patientenmanagementsystem und dem erfindungsgemäßen Verfahren zum Patientenmanagement ein System und ein Verfahren angegeben, wonach eine möglichst effiziente und wirksame Abwicklung des Patienten, insbesondere zur Unterstützung des Arztpraxisbetriebs, ermöglicht ist. Somit kann für den Arzt mehr Zeit verbleiben, um dem Patienten beispielsweise wichtige Zuwendung und Gespräche zuteilwerden zu lassen, die einen Fokus auf Gesunderhaltung und Stärkung der Ressourcen legen.

An dieser Stelle sei angemerkt, dass das Patientenmanagementsystem bzw. das computerimplementierte Patientenmanagementsystem als Softwaresystem auf einem leistungsfähigen Computer implementiert sein kann. Der Computer könnte in weiter vorteilhafter Weise über hochparallele Recheneinheiten in Form von leistungsfähigen Grafikeinheiten verfügen. Das Patientenmanagement kann modular ausgebildet sein, wobei ein Modul je nach Einsatzzweck entsprechende Berechnungen bzw. Berechnungsanalysen durchführen und - soweit erforderlich - auf etwaige verbundene Speicher und Datenbanken zugreifen kann. Ein Modul kann die Verwaltung und/oder Verwendung und/oder Verarbeitung von unterschiedlichsten Daten, insbesondere von patientenspezifischen Daten, bewerkstelligen. Dazu können in einem Modul entsprechende KI-basierte Algorithmen bzw. maschinelle Lernverfahren implementiert sein. Diese können auch die Verwendungen von neuronalen Netzen umfassen. Ferner können verschiedene Schnittstellenmodule den einfachen Zugang zu dem Patientenmanagement ermöglichen, beispielsweise über Web-basierte Protokolle.

In vorteilhafter Weise kann das Patienteninteraktionsmodul eine sichere und vertrauliche Interaktion der Patienten mit mehreren Interessensgruppen, vorzugsweise Ärzte und Behandlungsteam, über verschiedene Stadien der Interaktion hinweg ermöglichen. Beispielsweise können die Stadien eine Behandlung und/oder ein Erkrankungsverlauf eines Patienten umfassen.

In vorteilhafter Weise kann vorgesehen sein, dass in Abhängigkeit der (durch das Priorisierungsmodul ermittelten) Dringlichkeitsentscheidung eine Weiterleitung des Patienten im Patientenmanagementsystem bzw. zu einzelnen Modulen des Patientenmanagementsystems erfolgt, wobei die Weiterleitung eine Behandlungsempfehlung für den Patienten, vorzugsweise für verschiedene Stadien einer Behandlung und/oder eines Erkrankungsverlaufs, umfassen kann. In weiter vorteilhafter Weise kann in Abhängigkeit der Dringlichkeitsentscheidung eine Behandlungssteuerung des Patienten erfolgen. Somit kann ein Patientenmanagement bereitgestellt werden, das Patienten über einen gesamten Verlauf von Gesundheitsförderung über Konsultation bis zur Nachsorge digital kontinuierlich begleiten kann.

In vorteilhafter Weise kann das Patientenmanagementsystem ein Präferenzerfassungsmodul zur Ermittlung von Patientenpräferenzen anhand von gesammelten Nutzerdaten des Patienten umfassen. Die Nutzerdaten können dabei ein Nutzungsverhalten des Patienten hinsichtlich digitaler Dienste und/oder Medien, vorzugsweise aus dem Alltagsbereich, repräsentierten. Somit kann das Nutzungsverhalten des Patienten aus dem Alltag im Rahmen von Auswertungen, insbesondere basierend auf Algorithmen des maschinellen Lernens bzw. der künstlichen Intelligenz (KI), gewinnbringend für das Patientenmanagement berücksichtigt werden.

In weiter vorteilhafter Weise kann das Präferenzerfassungsmodul dazu ausgebildet sein, basierend auf den Nutzerdaten des Patienten einen Ist-Zustand und/oder Früh-Indikatoren im Hinblick auf ein geändertes Nutzungsverhalten des Patienten zu ermitteln. Somit kann dieses Wissen im Sinne der wissenschaftlichen Erkenntnisse zur Verhaltensveränderung in einem frühen Stadium der Veränderung zur gezielten Motivationsverstärkung eingesetzt werden. Ferner gibt das Benutzungsverhalten (Nachverfolgen von Links) eine frühe Indikation über mögliche Themenschwerpunkte, etwa zur Steuerung von Angeboten. Somit kann eine verbesserte Analyse über die Befindlichkeiten und den Gesundheitszustand des Patienten realisiert werden.

In einer vorteilhaften Ausgestaltung kann ein Telemedizinmodul vorgesehen sein, das zur Bereitstellung einer telemedizinischen Konsultation für den Patienten dient. Dabei ist denkbar, dass das Telemedizinmodul dem Patienten vorzugsweise dann bereitgestellt wird, wenn die Dringlichkeitsentscheidung des Priorisierungsmoduls dies erfordert. Das heißt, es erfolgt eine Weiterleitung des Patienten an ein Telemedizinmodul, wenn beispielsweise ein akutes bzw. dringliches Patientenanliegen vorliegt. In vorteilhafter Weise ist also eine Implementierung denkbar, so dass bei einer hohen Wahrscheinlichkeit für eine akute Behandlungsdringlichkeit, der Patient schnell in dem Patientenmanagementsystem weitergeleitet wird für die Kontaktaufnahme einer telemedizinischen ärztlichen Konsultation durch entsprechende (reale) Experten. Dabei kann die telemedizinische Konsultation unter Zugriff auf alle für die betroffenen Patienten vorhandenen patientenspezifischen Daten erfolgen. Somit kann gewährleistet werden, dass keine akute Problematik, welche einer dringlichen Behandlung bedarf, verpasst wird.

In einer vorteilhaften Ausgestaltung kann das Patienteninteraktionsmodul derart ausgebildet sein, dass weitere patientenspezifische Daten erfasst werden, sofern die Dringlichkeitsentscheidung des Priorisierungsmoduls dies zulässt. Die patientenspezifischen Daten können zweckmäßigerweise in einer Patientendatenbank als Patientenmerkmale hinterlegt werden.

In vorteilhafter Weise können die patientenspezifischen Daten Patientenmerkmale hinsichtlich der Ressourcen, der Präferenzen und/oder der Persönlichkeit des Patienten umfassen. Somit wird ermöglicht, dass im Rahmen einer patientenspezifischen Auswertung bzw. Analyse verbesserte Ergebnisse erzielbar sind.

In einer vorteilhaften Ausgestaltung kann die Erfassung der patientenspezifischen Daten mittels einem computeradaptiven Fragebogensystem durchgeführt werden. Dabei können die patientenspezifischen Daten zum Beispiel Informationen über Beschwerden und/oder Gesundheitszustände der Patienten umfassen. In vorteilhafter Weise können computeradaptive Fragebögen eingesetzt werden, welche eine ständige Interaktion mit einem entsprechenden KI-basierten erfordern. Durch diese vorteilhafte Ausgestaltung, bei welcher unter anderem Sachkenntnis zur Optimierung von Fragebögen mittels Item-Response-Theorie und/oder Bayes-Entscheidungs-Algorithmen zum Einsatz kommen kann, wird die Ausfallzeit für die Betroffenen gegenüber dem einfachen Einsatz der Originalfragebögen erheblich reduziert und führt so zu einer effizienten Datenerfassung.

In einer vorteilhaften Ausgestaltung kann das Patienteninteraktionsmodul derart ausgebildet sein, dass es mit einem mobilen Endgerät des Patienten interagiert, insbesondere um patientenspezifische Daten zu erfassen. Somit ist eine effiziente und einfache Datenerfassung für den Patienten realisierbar.

In einer vorteilhaften Ausgestaltung können über eine Sensoreinrichtung des mobilen Endgeräts patientenspezifische Messdaten erfasst werden. Die patientenspezifischen Messdaten werden als Patientenmerkmale berücksichtigt und im Rahmen von Bewertungen und Analysen in den Bezug auf den Patienten verwendet. Zweckmäßigerweise werden die Messdaten in einer Patientendatenbank hinterlegt bzw. gespeichert.

In einer vorteilhaften Ausgestaltung kann das Patientenmanagementsystem ein Auswertungsmodul umfassen, wobei das Auswertungsmodul dazu ausgebildet ist, basierend auf den patientenspezifischen Daten bzw. den Patientenmerkmalen, eine Zustandsbeschreibung des Patienten zu erzeugen. In vorteilhafter Weise sind zur Bestimmung der Zustandsbeschreibung KI-basierte Algorithmen bzw. maschinelle Lernverfahren implementiert, so dass eine umfassende Auswertung von patientenspezifischen Daten bzw. Patientenmerkmalen durchgeführt werden kann.

In vorteilhafter Weise kann basierend auf der Zustandsbeschreibung des Patienten ein Matching bzw. ein Abgleich mit vorgegebenen Merkmalen einer Arztpraxis durchgeführt wird, um eine Abwicklung des Patienten in der Arztpraxis zu steuern. Somit kann beispielsweise eine effiziente Terminbuchung in der Arztpraxis realisiert werden, wobei sowohl das Anliegen des Patienten als auch die verfügbaren Ressourcen in der Arztpraxis auf raffinierte Weise Berücksichtigung finden können.

In einer vorteilhaften Ausgestaltung kann das Patientenmanagementsystem mindestens ein Visualisierungsmodul zur Bereitstellung von Informationen an den Patienten und/oder an den Arzt bzw. das Praxisteam umfassen. Über das Visualisierungsmodul können - beispielsweise im Vorfeld der Arztkonsultation - dem Patienten bereits umfassende Informationen an die Hand geben.

Des Weiteren kann das Visualisierungsmodul als Behandlungsbegleitmodul ausgebildet sein, wodurch - beispielsweise im Zusammenwirken mit dem Patienteninteraktionsmodul und/oder dem Auswertungsmodul - der Arzt bzw. das Behandlungsteam in einer Behandlungsentscheidung unterstützt werden kann. Dabei ist denkbar, dass individualisierte und/oder auf die spezifischen Präferenzen der Patienten abgestimmte Diagnostik und/oder Vorschläge zur Prävention, Gesundheitsförderung, Indikationsstellung, Behandlung und/oder Weiterleitung bereitgestellt werden können. Zur Gewinnung bzw. Erzeugung dieser Informationen kann beispielsweise auf das Auswertungsmodul bzw. auf dortige Algorithmen, Berechnungen und/oder Analysen zurückgegriffen werden. Ferner kann durch das Visualisierungsmodul auch eine effiziente Kommunikation und Wissensvermittlung im Arztgespräch zwischen Arzt und Patient ermöglicht werden.

Schließlich kann das Visualisierungsmodul auch im Rahmen der Nachsorge als Nachsorgemodul Verwendung finden und so dem Patienten auf effiziente Weise weitergehende Information und ggf. Anweisungen bereitstellen. In vorteilhafter Weise kann das Visualisierungsmodul als Nachsorgemodul ausgebildet sein, das - nach einer Arztkonsultation - zur Bereitstellung von Informationen an den Patienten dient. Das Nachsorgemodul kann zum Beispiel dazu ausgebildet sein, vorzugsweise im Zusammenwirken mit dem Patienteninteraktionsmodul, eine begleitende und durch digitale Dienste zuverlässige und verbesserte Nachsorge zu ermöglichen. So kann in vorteilhafter Weise auch bei unerwarteten Zustandsverläufen des Patienten eine raschere Anpassung der Behandlung ermöglicht werden.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht eine Veranschaulichung des Potentials hinsichtlich einer möglichen Verbesserung für den Patienten durch ein Ausführungsbeispiel der Erfindung gegenüber einem bisherigen Vorgehen aus dem Stand der Technik,
- Fig. 2: in einer schematischen Ansicht einen beispielhaften Prozessablauf für einen Patienten durch einzelne Ebenen eines Patientenmanagementsystems gemäß einem Ausführungsbeispiel der Erfindung, und
- Fig. 3: in einer schematischen Ansicht einen beispielhaften Ablauf eines Patientenmanagementsystems bzw. eines Verfahrens zum Patientenmanagement gemäß einem Ausführungsbeispiel der Erfindung.

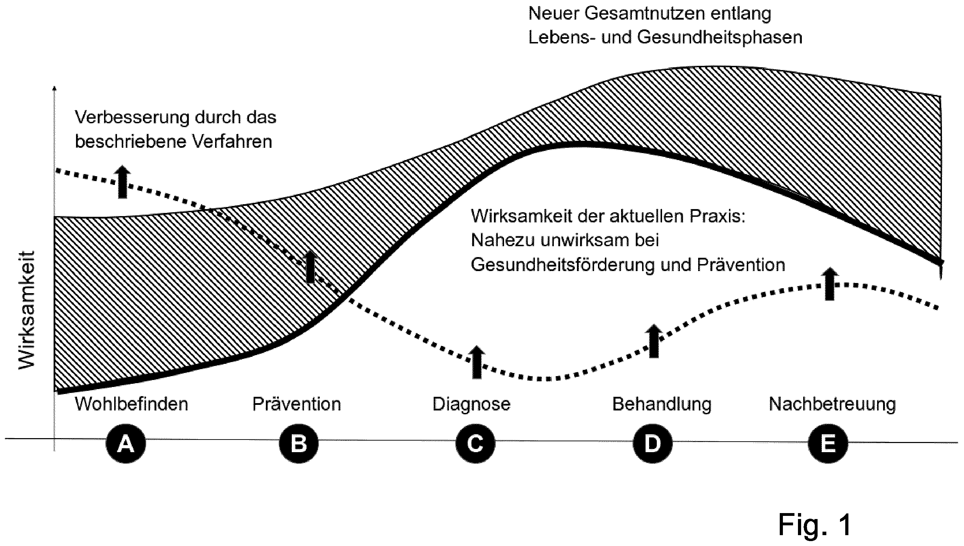

Fig. 1 zeigt in einer schematischen Ansicht eine Veranschaulichung des Potentials hinsichtlich einer möglichen Verbesserung für den Patienten durch ein Ausführungsbeispiel der Erfindung gegenüber einem bisherigen Vorgehen aus dem Stand der Technik.

Das Patientenmanagementsystem bzw. Verfahren zum Patientenmanagement gemäß einem Ausführungsbeispiel der Erfindung kann zum Betrieb von Gesundheitspraxen, Praxen der Hausarztversorgung, Gemeinschaftspraxen oder medizinischen Versorgungszentren in der Allgemeinmedizin oder hausärztlichen Versorgung dienen.

Dabei ist das System bzw. das Verfahren gemäß einem Ausführungsbeispiel der Erfindung derart ausgelegt, dass es in einer neuartigen und effizienten Weise die Prozesse strukturiert: dies umfasst ein Verfahren von der ersten Kontaktaufnahme, Erheben der Vorgeschichte, Beschwerden und Befunde über die eigentliche persönliche Behandlung bis hin zur Nachsorge und Erfolgskontrolle unter Einbezug digitaler Technologien (z.B. mobile Endgeräte, digitale Gesundheitsanwendungen), Referenzdatenbanken, durch maschinelles Lernen (künstliche Intelligenz) unterstützten Expertensystemen, integrierter telemedizinischer Versorgung und auf die neuen Behandlungsprozesse spezifisch eingerichteten Praxisräumlichkeiten.

Ein durch Ausführungsformen der Erfindung ermöglichte neue hybride, d.h. digital und analog kombinierte, und an den individuellen Bedarfen und Präferenzen der Patienten ausgerichtete Vorgehen erlaubt ferner, über gezielte Unterstützung, insbesondere durch Einsatz digitaler Technologien (z.B. mobile Endgeräte) und digitale Biomarker (z.B. Erkennung von Erkrankungen aus Sprachdaten) eine Gesundheitsförderung und Prävention personalisiert in den Behandlungsprozess zu integrieren. So generiert das Vorgehen bei vergleichbaren Kosten zur heutigen Praxis langfristig überlegene Ergebnisse (Verbessertes Kosten-Nutzen-Verhältnis) und einen stark verbesserten Nutzen für die Patienten und die Health Professionals.

Ein Ausführungsbeispiel der Erfindung kann ein Verfahren zum patientenzentrierten Management und den Betrieb von Praxen in der Allgemeinmedizin oder hausärztlichen Versorgung umfassen. Im Vergleich zur derzeitigen Lehre kann unter Einsatz mobiler Endgeräte und digitaler Technologie, Referenzdatenbanken, durch künstliche Intelligenz unterstützte Expertensysteme, telemedizinischen Verfahren und/oder auf die neuen Behandlungsprozesse spezifisch eingerichtete Praxisräumlichkeiten der gesamte Behandlungsprozess von der ersten Kontaktaufnahme bis zur Nachverfolgung und Erfolgskontrolle auf geschickte Weise als hybrider Prozess - d.h. als Kombination von analogen (= persönlicher Kontakt) und digitalen Lösungen unter Einsatz von IT-Technologien, digitalen Biomarkern und computergestützten Expertensystemen - vollkommen neu strukturiert werden. Ausführungsformen der Erfindung richten sich - im Gegensatz zur bisherigen Lehre nach dem Stand der Technik - an den individuellen Präferenzen und Konstellation der einzelnen Patienten aus und vermag so im Gegensatz zur bisherigen Lehre auch Potenziale von Gesundheitsförderung und Prävention in einer personalisierten Form auszuschöpfen.

Ausführungsbeispiele der Erfindung bringen insbesondere bei der Gesundheitsförderung/Wohlbefinden, der Prävention und der Nachbetreuung einen entscheidenden Zugewinn, wie dies in Fig. 1 veranschaulicht ist.

Erschwerend für die derzeitige Lehre nach dem Stand der Technik kommt hinzu, dass die derzeitigen Arbeitsbedingungen für angehende Ärzte unattraktiv sind und gerade auf dem Land viele hausärztliche Sitze bereits heute keine Nachfolge finden. Auch diese Herausforderung kann durch ein Ausführungsbeispiel der Erfindung adressiert, indem es nämlich einen ganz anderen Betrieb ermöglicht, der in Bezug auf den einzelnen Arzt bedeutsam mehr Zeit für die wirklich relevanten Patientenkontakte herausfiltriert, vorzugsweise durch geschickte Prozessoptimierung und unterstützt durch digitale Medizinprodukte. Ferner erlaubt eine Ausführungsform der Erfindung sehr viel flexiblere Arbeitsmodalitäten für das Personal, eine Forderung gerade von jungen Ärzten, aber auch von medizinischem Fachpersonal. Grundlage für eine Ausführungsform der Erfindung ist der kombinierte Einsatz von Technologie, darauf spezifisch abgestimmten Räumlichkeiten und Raumkonzepten, ggf. unter Nutzung von nonverbalen Ausprägungen, wie etwa Lichtgestaltung, Hintergrundmusik und Düften sowie dem konsequenten Einsatz von künstlicher Intelligenz und digitalen Biomarkern zur Unterstützung der Behandlungsführung.

Fig. 1 zeigt einen Vergleich der bisherigen Lehre und Praxis (durchgezogene Linie in Fig. 1) in Bezug auf die Wirksamkeit in den einzelnen Gesundheits- und Lebensphasen. Das Potenzial, das durch Ausführungsformen der Erfindung geschöpft werden kann ist in Fig. 1 durch die gestrichelte Linie dargestellt, wobei mit deutlich gesteigertem Gesamtnutzen (obere Begrenzung der schraffierten Fläche) in allen Gesundheitsphasen, insbesondere aber bei Gesundheitsförderung bzw. Wohlbefinden, Prävention und Nachbetreuung.

Fig. 1 verdeutlicht den möglichen Zugewinn entlang der Gesundheit oder Lebensphasen der Betroffenen, Kunden oder Patienten. Die bisherige hausärztliche Versorgung fokussiert hingegen vor allem auf die durch die durchgezogene Linie gezeigte Exzellenz in der Diagnose und unmittelbaren Behandlung. Dies wird durch Vergütungssysteme in den unterschiedlichen Gesundheitssystemen verstärkt, legitimiert, sanktioniert und honoriert. In den meisten Gesundheitssystemen ist das Verhalten überwiegend angebotsorientiert, d.h. es wird eine maximale Auslastung der Leistungsangebote angestrebt.

Ausführungsformen der Erfindung sind im Kontext aktueller gesellschaftlicher Makrotrends zu sehen, da diese unterstreichen, warum Ausführungsformen der Erfindung bislang nicht möglich waren. Es existieren lediglich diverse Einzellösungen, etwa digitale Rezepte oder telemedizinische Sprechstunden, jedoch sind all diese Einzellösungen bislang nicht zu einem kohärenten Betriebssystem für die hausärztliche Praxis der Zukunft zusammengefügt und intelligent vernetzt worden. So ist die Lehre nach dem Stand der Technik davon gekennzeichnet, dass eine unzusammenhängende Kakophonie neuer technologischer digitaler Gesundheitsanwendungen auf ein traditionell analog strukturiertes System trifft und so das Gesamtsystem eher verkompliziert als vereinfacht. Auch von der neu eingeführten digitalen Patientenakte ist daher kein wesentlicher Fortschritt zu erwarten.

Im Rahmen von Ausführungsformen der Erfindung ist daher erkannt worden, dass von besonderem Vorteil ist, wenn der gesamten Prozess als "Customer Journey" verstanden wird, mit sich im Verlauf der Customer Journey im Gegensatz zu anderen Wirtschaftssektoren auch wandelnden Bedürfnissen (von selbstbewusster aufgeklärter Konsument zum hilfesuchenden Notfallpatienten in der gleichen Person) und dies konsequent unter Ausnutzung von im Nachfolgenden spezifizierten Technologien, Geräten, räumlichen Ausprägungen und im Hinblick auf zukünftige Anwendungen künstlicher Intelligenz sowie die Anforderungen eines konsequenten Datenschutzes in ein zuverlässig funktionierendes Verfahren eines hybriden analogdigitalen Betriebssystems für eine neue hausärztliche Versorgung zu bündeln.

Der gesellschaftliche Makrotrend, auf dessen Hintergrund Ausführungsformen der Erfindung aufbauen, lässt sich wie folgt zusammenfassen: Gesundheit durchzieht als Leitmotiv zunehmend alle Lebenswelten - man spricht von gesunden Finanzen, sich gesund zu ernähren, von gesunder Arbeit, von gesund altern und gesund wohnen bis zu gesunder Umwelt. Der Fokus verschiebt sich dabei immer weiter weg von der Krankheit auf gestaltbare und erlebbare Gesundheit, für jeden Menschen an jedem Tag. Gesundheit wird gleichzeitig für immer breitere Kreise zum Lifestyle. Mit der Verschiebung weg von Krankheit und hin zu einer gesunden Lebensweise verändert sich auch die Erwartungshaltung der Menschen zu Gesundheit. In anderen Wirtschaftssektoren setzen Kundinnen und Kunden als "Customer" heute voraus, dass eine Dienstleistung möglichst digital, sofort und transparent verfügbar ist. Wer es zuerst schafft, diese veränderte Erwartungshaltung der Menschen aufzugreifen und in einem neuen, stärker auf Gesundheit ausgerichteten Ökosystem die "Consumer Journey" ins Zentrum der Prozesse zu stellen, wird das Vertrauen der Menschen gewinnen. Hier wird eine Neuausrichtung der Hausarztmedizin auf Gesundheitsförderung und Prävention - unterstützt durch Digitalisierung - Orientierung schaffen und gesundheitlichen Mehrwert anbieten, dem die Bürgerinnen und Bürger vertrauen. Gerade junge Menschen, die sich über Social Media austauschen, denken nicht in erster Linie an ihren Arzt, wenn es um Gesundheitsthemen geht.

Ausführungsformen der Erfindung ermöglichen durch die Digitalisierung, viele Fragestellungen und Abklärungen einschließlich Gesundheitscoaching in einen digitalen Raum vor den Praxisbesuch zu verlagern. Sie können in Einzelfällen durch vereinfachten Datenaustausch und Nutzen telemedizinischer Konsultation von Patient zu Facharzt oder Hausarzt zu Facharzt das tatsächliche Aufsuchen der Spezialisten erübrigen. Verbesserte Algorithmen können die ärztliche Kunst der Differenzialdiagnose weiter erleichtern. Telemedizinische Konsultationsdienste in Verbindung mit künstlicher Intelligenz und verbesserten Algorithmen, die auch den Verlauf von Gesundheit bzw. Krankheiten berücksichtigen, können das Aufsuchen der Hausarztpraxis bei nichtchirurgischen Problemen weitgehend erübrigen.

Das Betriebsverfahren für eine Praxis, die den gesellschaftlichen Makrotrend zu mehr Gesundheit aufgreift, hat neben einer konsequenten Nutzung der dargelegten Digitalisierungschancen stärker als bisher auf gesundheitliche Ressourcen und Wohlbefinden ausgerichtet zu sein. Denn hier liegt das größte unausgeschöpfte Potenzial im Hinblick auf einen verbesserten Endnutzen (Outcomes), sei es etwa durch Verhaltensänderung, durch Stärken der psychischen Gesundheit oder Verbessern der Gesundheitskompetenz.

Ein Patientenmanagementsystem gemäß einem Ausführungsbeispiel der Erfindung kann sämtliche digital verfügbaren Register ziehen, um im Zusammenwirken mit einer den Überblick behaltenden Vertrauensperson - dem neu erfundenen alten Hausarzt - eine Begleitung vor allem auf dem Lebensweg zu mehr Gesundheit und auch bei gelegentlicher Krankheit sein.

Diese Vorgehensweise als neue Form einer Hausarztpraxis kann durch digitale Lösungen in geschickter Kombination mit real existierenden Menschen - etwa aus dem bürgerschaftlichen Engagement - auch viel besser in das gesamte Teilhabeund Versorgungsmanagement von älteren Menschen mit chronischem Unterstützungsbedarf integriert werden. Eine solche, primär auf das Wohlbefinden in jedem Lebensalter ausgerichtete Gesundheitspraxis kann auch der Gesundheit zuträgliche Verknüpfungen zu anderen Wertschöpfungsbereichen oder zu anderen kommunalen Ressourcen ermöglichen, wenn das Vorgehen und die Entscheidungen transparent bleiben. Im Zentrum steht - im Gegensatz zu rein digitalen oder telemedizinischen Lösungen - unverändert die reale, persönliche und vertrauensvolle Beziehung zu Ärzten oder zu anderen für die jeweilige Fragestellung optimal ausgebildeten Fachpersonen.

Ein wesentlicher Aspekt von Ausführungsformen der Erfindung ist es, durch das Nutzen digital unterstützter Technologie, die Zeit für eine reale Beziehung in der Praxis freizuspielen und viele bislang noch analoge Arbeitsschritte - etwa das Erheben der Krankengeschichte - in den digitalen Raum zu verlagern oder als mittels digitaler Lösungen supervidierte und qualitätskontrollierte Arbeit in das Praxisteam zu delegieren. So bleib bei einer Anwendung einer Ausführungsform der Erfindung trotz Knappheit der ärztlichen Arbeitskraft mehr Zeit für das, was Vertrauen und Beziehung schafft: das zuwendende motivierende Gespräch mit den Patienten. Verfolgt eine Arztpraxis die angesprochene Ausrichtung auf die "Consumer Journey", wird sie auch von Menschen aufgesucht, die bislang den Gang zur Arztpraxis eher meiden.

Ausführungsformen der Erfindung lösen die zuvor genannten Probleme durch geschicktes Zusammenführen in einem neuartigen Verfahren zum Betrieb eines hausärztlichen Versorgungszentrums, welches in neuartiger Weise digitale Lösungen, Expertensysteme, vorhandene Sensoren und Eingabegeräte sowie innovative Ausprägung und Anwendung von innenarchitektonischen Lösungen mit konsequenter, durch künstliche Intelligenz unterstützender Prozessgestaltung kombinieren kann. Eine konsequente Umsetzung erhöht entlang der Customer Journey die Wahrscheinlichkeit von Verhaltensänderung und Compliance. Die konsequente Begleitung der Patienten durch Erhebung und Auswertung von Messwerten und Abfragen der subjektiven Beschwerden und Erfolge ermöglicht weit bessere Outcomes für Patienten als es bisher in der Versorgung möglich war. Auf diese Weise kann ein Patientenmanagementsystem gemäß einem Ausführungsbeispiel der Erfindung als Betriebssystem bei aktuell gleichen Behandlungskosten langfristig zu einer Reduzierung der Folgekosten beitragen und erhöht damit die volkswirtschaftliche Wirksamkeit bzw. den volkswirtschaftlichen Nutzen.

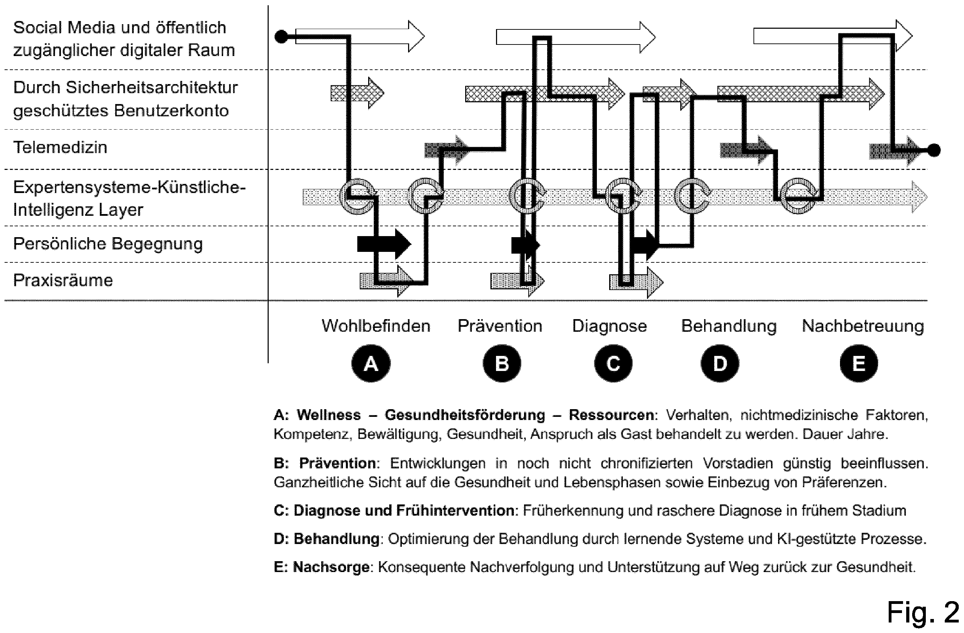

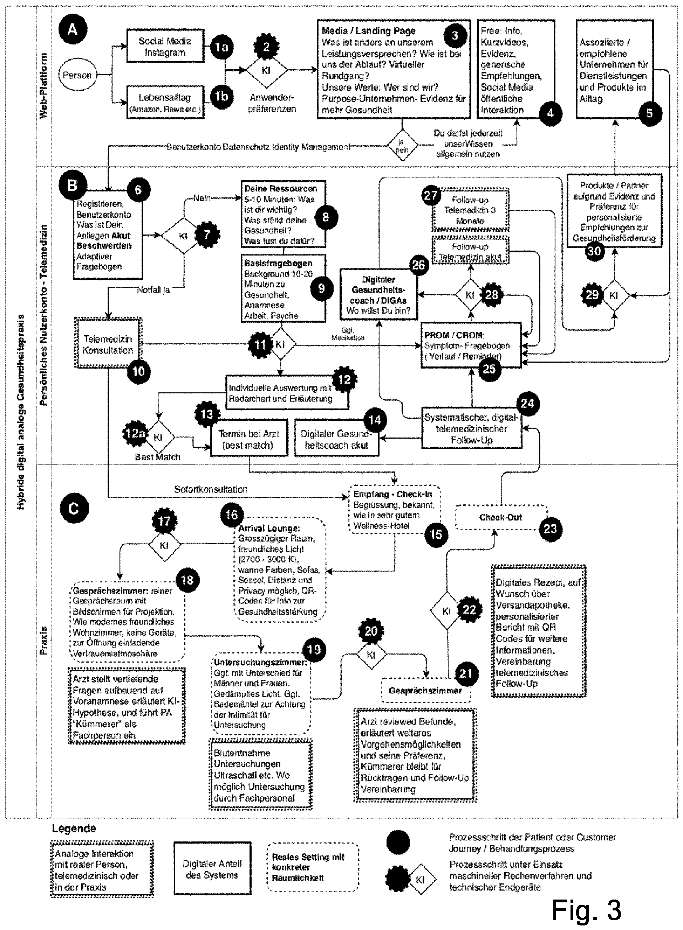

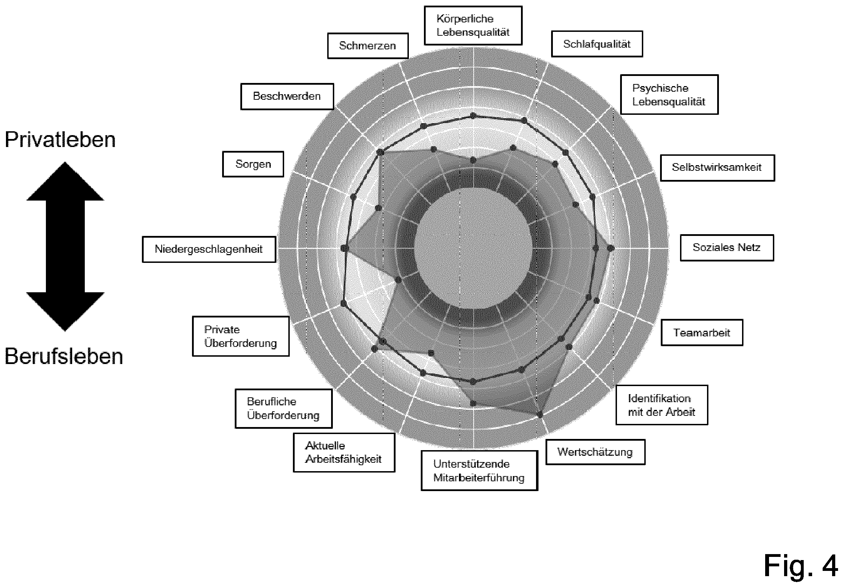

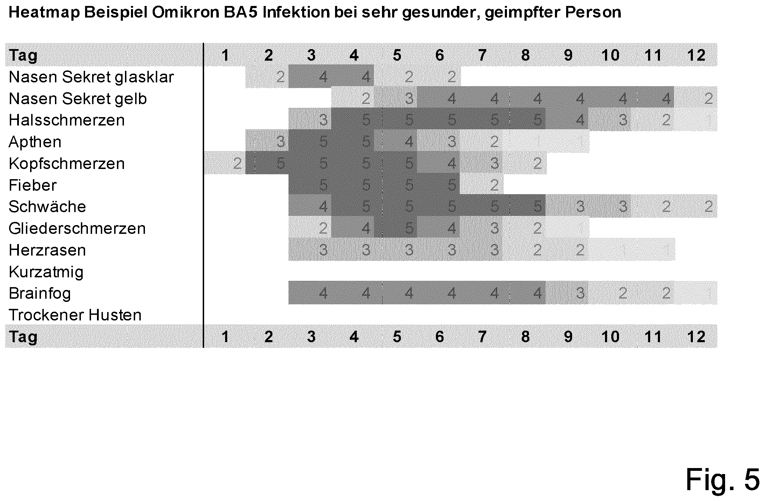

Ein Patientenmanagementsystem gemäß einem Ausführungsbeispiel der Erfindung als Betriebssystem zukünftiger hausärztlicher Versorgung kann als wesentlichen Aspekt umfassen, dass eine konsequente Ausgestaltung des Prozesses der Customer Journey in der Anwendung von Innovationen in zwei digitalen und einem analogen Bereich vorgesehen ist. Beispielsweise so, wie dies in Fig. 2 und Fig. 3 veranschaulicht ist.

Fig. 2 zeigt in einer schematischen Ansicht einen beispielhaften Prozessablauf für einen Patienten durch einzelne Ebenen eines Patientenmanagementsystems gemäß einem Ausführungsbeispiel der Erfindung. Im Konkreten zeigt Fig. 2 eine exemplarische Customer-Journey durch die integrierten Ebenen von öffentlich zugänglichen Datenräumen über geschützte Datenräume nach GAIA-X Standard, kontinuierliche Evaluation durch Expertensysteme / künstliche Intelligenz verknüpft mit gezieltem Einsatz von Telemedizin sowie persönlicher Konsultation in entsprechend eingerichteten Räumlichkeiten.

Ein Patientenmanagementsystem gemäß einem Ausführungsbeispiel der Erfindung kann ein Gewebe aus verschiedenen digitalen Lösungen umfassen, welche einerseits auf mobile Endgeräte zurückgreifen und die darin eingebaute Sensorik, andererseits auf spezifische Endgeräte, in Verbindung mit Algorithmen, selbst lernenden Expertensystemen und durch entsprechende Endgeräte unterstützte Visualisierungen von in Rechenzentren ausgewerteten Datenströmen, dies unter Einhaltung aller Vorschriften und Vorkehrungen der Datensicherheit geschickt mit der Interaktion mit real existierenden Fachpersonen, seien es Ärzte oder spezifisch ausgebildetes Fachpersonal, verknüpft.

Durch die Nutzung von digitalen Endgeräten auf Patientenseite, wie auch die Interaktion mit künstlicher Intelligenz nicht nur in partikularen Einzelfragestellungen, entsteht nunmehr die Möglichkeit, Betroffene über die gesamte Lebensspanne und den gesamten Bedarf von Wohlbefinden über Prävention, frühe Diagnose, gezielte und rechtzeitige Behandlung bis zur effizienten Nachsorge einzubeziehen. Dadurch können die theoretisch und in vielen einzelnen wissenschaftlichen Studien nachgewiesenen Potenziale in der Praxis viel besser ausgeschöpft werden.

Ein Verfahren zum Patientenmanagement gemäß einem Ausführungsbeispiel der Erfindung kann durch regelkreisartige Feedback-Schleifen über die verschiedenen Systemkomponenten hinweg seine prognostische Stärke gewinnen, die in einer vergleichbaren Weise heute bereits etwa in den durch künstliche Intelligenz unterstützten Verfahren zur Überwachung von Triebwerken oder anderen kritischen Bauteilen in sicherheitsrelevanten technischen Prozessen zur Anwendung möglich sind. Gemäß einer Ausführungsform der Erfindung wird dies auf die Prognose und Steuerung von Verhaltensentwicklung, Gesundheitsentwicklung, Stärkung von persönlichen Ressourcen, Krankheitsverläufe und Heilung erweitert. Dies entlastet das ärztliche und nicht-ärztliche Personal, erhöht die Sicherheit und die Behandlungsqualität. Dabei können Erkenntnisse aus der Verhaltenspsychologie und Verhaltensökonomie eingesetzt werden, etwa auch bei der Ausgestaltung der Räumlichkeiten. Ein besonderes Merkmal ist die stete Erfassung von patientenspezifischen Daten der Patienten über digital präsentierte Fragebögen, Erinnerungsfunktionen und dies verknüpft mit den klinischen Daten über Expertensysteme und computergestützte künstliche Intelligenz, so dass in der hausärztlichen Versorgung auch ein konsequentes Prozess-Mining möglich wird.

Ein besonderer Vorteil eines Patientenmanagementsystems bzw. eines entsprechenden Verfahrens gemäß einem Ausführungsbeispiel der Erfindung ist, dass erstmals bereits in der Technik für rein deterministisch beschreibbare Systeme wie etwa für Triebwerke anwendbare Verfahren auf nicht deterministisch beschreibbare Systeme wie den gesunden Menschen mit gelegentlicher Erkrankung angewendet werden können. Dabei kann der Mensch konzeptionell als ein durch Methoden der Chaos-Theorie beschreibbares System verstanden werden, das eingebettet ist in seine individuellen biologischen, genetischen und physischen Determinanten, wie auch die Determinanten auf der Meso-Ebene, namentlich die wirtschaftlichen und sozialen Bedingungen, unter denen Menschen geboren werden, leben, arbeiten und altern.

Ein Patientenmanagementsystem als Betriebssystem für eine begleitende hausärztliche Gesundheitspraxis, die den Anspruch erhebt, die Betroffenen bzw. Patienten über alle Gesundheitsphasen und entlang des Lebensweges zu begleiten, kann daher in vorteilhafter Weise mehrere verschiedene Elemente und Erkenntnisse berücksichtigen und deren Zusammenwirken sinnvoll unterstützen, zum Beispiel:
- Einbringen von Erkenntnissen aus Verfahren der Klimaforschung und/oder der prognostisch adaptiven Wartung von komplexen technischen Systemen, insbesondere in die Betreuung von (vorzugsweise gesunden) Personen;
- Nutzung von verfügbaren technologischen Möglichkeiten der Sensorik in mobilen Endgeräten;
- Nutzung der Darstellungsmöglichkeiten von Information auf mobilen Endgeräten,
- Nutzung der Interaktionsmöglichkeiten mit Personen;
- Nutzung der sicheren Verknüpfung von dezentral gelagerten Daten auf entsprechend ausgestatteten Rechenzentren;
- Nutzung von adaptiven an die Individualität angepassten Lichtstimmungen in einem Praxisraum; und/oder
- Nutzung einer durch künstliche Intelligenz gefundenen und für das Individuum möglichst nützlichen Kombination von Musik, vorzugsweise konzertiert eingesetzt im Zusammenwirken mit einer persönlichen Interaktion mit einer vertrauenswürdigen Fach-Person.

Im Zentrum dieses Prozesses kann die bislang vollkommen vernachlässigte und durch mobile Endgeräte erleichterte wiederholte Befragung der Betroffenen stehen, nämlich beispielsweise nach ihren Präferenzen, nach ihren Ressourcen, nach akuten Beschwerden und/oder nach möglichen anderen Anliegen rund um Gesundheit. Dies alles kann durch Einsatz adaptiver Verfahren erfolgen, welche in der Einzelbefragung den Aufwand für die Betroffenen minimieren, vorzugsweise durch den Einsatz von Algorithmen, selbst lernenden Systemen und in einer Ausprägung künstlicher Intelligenz den Informationsgewinn maximieren.

Ferner können in Kombination mit den bereits heute verfügbaren klinischen Daten, Laborparametern und Verhaltensindikatoren unter Einsatz von unter hohen Sicherheitsvorkehrungen dezentral betriebenen Rechenzentren Informationsverdichtungen entlang der gesamten Customer Journey entstehen. Auf diese Weise können personalisierte und damit hochgradig individualisierte Empfehlungen ermöglicht werden, angefangen von der Steigerung des Wohlbefindens, über Prävention oder Früherkennung von noch nicht manifesten Erkrankungen, Differenzialdiagnose bei unklaren Beschwerden bis hin zur Nachsorge die Betreuungsqualität zu steigern. Es liegt umfassende wissenschaftliche Evidenz vor, dass ein derartiges Vorgehen selbst bei terminalen Erkrankungen wie einem metastasierendem Lungenkarzinom die Outcomes massiv verändern kann. So wurde nur durch die Berücksichtigung des individualisiert abgefragten Befindens der Patienten die Überlebenszeit dieser Personen in einer randomisierten Studie um 50 % von 12 auf 18 Monate gesteigert.

Ausführungsformen der Erfindung ermöglichen, dass möglichst viele der heute entweder gar nicht stattfindenden aber notwendigen Anteile der Customer-Journey in den digitalen Raum verlagert werden und damit erstens skalierbar und zweitens einer maschinellen Bearbeitung zur Unterstützung von Entscheidungsfindung zugänglich gemacht werden können. Dies erfordert das sorgfältige Zerlegen der heute in der ärztlichen Konsultation im Sprechzimmer stattfindenden Komponenten im Hinblick darauf, was bereits im Vorfeld unter Einsatz digitaler Endgeräte in der Alltagsumgebung der Patienten erhoben, bearbeitet, vermittelt werden kann. Ein Patientenmanagementsystem gemäß einer Ausführungsform der Erfindung kann dabei auf verschiedenste Behandlungsanlässe angewendet werden, die in Klassen von Anlässen kategorisiert sind. Beispielsweise findet heute die von der gesetzlichen Krankenkasse vergütete Gesundheitsuntersuchung als persönlich erbrachte ärztliche Leistung statt.

Beispielsweise kann allein eine vorgeschriebene Erhebung der Krankengeschichte und psychischen Situation bei sorgfältiger Ausführung wenigstens 20-25 Minuten benötigen. Ebenso benötigt die in der Regel durchzuführende Präventionsberatung mindestens 15 bis 20 Minuten, werden auch psychische Fragestellungen mit einbezogen. Im Rahmen eines Ausführungsbeispiels der Erfindung kann die Erhebung der Krankengeschichte bereits vor der Konsultation stattfinden, beispielsweise durch adaptive Fragebögen, vorzugsweise auf mobilen Endgeräten. Vor der Konsultation kann die Person dann bereits ein personalisiertes Feedback erhalten, das beim persönlichen Kontakt in entsprechenden Räumlichkeiten visualisierbar ist. Auch eine Aufklärung zur Nachsorge und/oder eine Begleitung durch den möglichen Veränderungsprozess kann in den digitalen Raum skalierfähig ausgelagert werden und durch entsprechend allgemein zugängliche Inhalte in Social Media noch motivational verstärkt werden. Eine Anbindung an ein persönliches Benutzerkonto des Patienten erlaubt neue Verbindungen zu anderen Wirtschaftssektoren, etwa personalisierte Kaufempfehlungen für den Einkauf von Lebensmitteln. Bei entsprechender Zustimmung der Betroffenen (Datenschutz) ist im Rahmen eines Ausführungsbeispiels der Erfindung denkbar, dass derartige personalisierte Verkaufsempfehlungen zu gesundheitsförderlichen Lebensmitteln während des Einkaufs im Lebensmittelhandel über Augmented Reality auf mobilen Endgeräten eingespielt werden.

Fig. 3 zeigt in einer schematischen Ansicht einen beispielhaften Ablauf eines Patientenmanagementsystems bzw. eines Verfahrens zum Patientenmanagement gemäß einem Ausführungsbeispiel der Erfindung. Dabei wird veranschaulicht, wie das in Fig. 2 dargestellte "Gewebe" der Interaktion von persönlichem Zusammentreffen mit einer Fachperson, individualisierten digitalen Lösungen oder telemedizinischen Angeboten, regelmäßiger Befragung, Erfassen von Zuständen mittels digitaler Endgeräte, unterstützende digitale oder hybride (d.h. analog und digital) Gesundheitslösungen und allgemein verfügbare Information in einem konkreten Ausführungsbeispiel der Erfindung mit einzelnen Komponenten realisiert und praktisch umgesetzt werden kann:

### A: Öffentlich zugänglicher digitaler Bereich

**1. Social Media und Lebensalltag:** Durch entsprechende Links oder Bonuskartensysteme bei Einkäufen kann, sofern das Einverständnis der Betroffenen vorliegt, ihr Benutzungsverhalten mit einbezogen werden, um daraus einerseits den aktuellen Ist-Zustand, vor allem aber Früh-Indikatoren für geändertes Benutzungsverhalten zu gewinnen. Dieses Wissen kann im Sinne der wissenschaftlichen Erkenntnisse zur Verhaltensveränderung in einem frühen Stadium der Veränderung zur gezielten Motivationsverstärkung eingesetzt werden. Ferner gibt das Benutzungsverhalten (Nachverfolgen von Links) eine frühe Indikation über mögliche Themenschwerpunkte, etwa zur Steuerung von Angeboten.
**2. Ermittlung der Patientenpräferenzen aus der Nutzung von Social Media und Verhalten der Patienten im Lebensalltag, z.B. durch Smart-Devices mit maschinellem Lernen:** Die unter 1. gewonnenen Informationen zu Nutzerdaten müssen streng getrennt von anderen, gesundheitsbezogenen und besonders schützenswerten Daten verarbeitet werden. Daher ist hier eine eigenständige Instanz der Analyse vorgesehen, welche neuronale Netze oder andere Verfahren der künstlichen Intelligenz einsetzt, Muster früh zu erkennen. Diese digitale Instanz analysiert auch das Benutzungsverhalten auf den öffentlich zugänglichen digitalen Angeboten der Gesundheitspraxis.
**3. Landing Page, Website und virtueller Rundgang:** Auf der Website werden die Leistungsversprechen der Gesundheitspraxis vorgestellt. Zudem wird der Ablauf sowohl digital virtuell und analog vorgestellt. In Form eines virtuellen Rundgangs können sich die späteren Patienten einen Einblick in die Arbeit und die physischen Strukturen der Praxis verschaffen. Die Nutzer werden vertraut mit den Werten und der Gesamtkonzeption von Gesundheit und Ressourcen stärken auf Basis wissenschaftlicher Evidenz. Informationen und Kurzvideos über den Ablauf der Customer Journey in den Praxen können hier zur Verfügung stehen. Dies dient unter anderem dem Erwartungsmanagement zukünftiger Praxisbesucher, etwa der Notwendigkeit vorab, d.h. vor der Konsultation, wesentliche Informationen über digital adaptive Fragebögen zur Verfügung zu stellen und einen aufgrund dieser Vorinformationen gezielt ausgewählten Termin zu buchen.
**4. Evidenzbasierte vertiefende Information:** Kurzvideos und vertiefende Informationen zu wichtigen Themen, insbesondere gesundheitsstärkenden Ressourcen wie Schlaf, Ernährung, Selbstwirksamkeit, Wertschätzung, Berührung, Freundschaft aber auch zu komplexen Themen wie Nachhaltigkeit und Gesundheitsverhalten. Der Fokus liegt auf dem Stärken von Gesundheitskompetenz. In mehr Infotainment-Charakteristik wird das Wissen komprimiert und jedermann jederzeit zugänglich auch auf Social Media dargeboten. Diese digitalen Angebote sollen eine Community schaffen, die eine Bindung zur Gesundheitspraxis aufbaut, auch wenn gar kein Krankheitsanliegen vorliegt. Auf diese Weise können Wertschöpfungspotenziale erschlossen werden, die sich auf Gesundheit und Schönheit beziehen und bei denen Kundinnen und Kunden von vornherein akzeptieren, dass sie nicht durch die Leistungen der gesetzlichen Krankenversicherung abgedeckt sind.
**5. Sektorübergreifende Verknüpfung zu gesundheitsförderlichen Dienstleistungen und Produkten:** In einem Ausführungsbeispiel kann vorgesehen sein, dass gezielt Verbindungen zu Einkaufsquellen etwa für Nahrungsergänzungsmittel, bestimmte gesundheitsförderliche Nahrungsmittel oder aber auch Lebensmitteldiscounter (z. B. Rewe) aufgebaut werden, gegebenenfalls über Augmented Reality. Der Nutzen hier für die Patienten bzw. Kunden ist einerseits die evidenzbasierte Empfehlung, andererseits ein möglicher Rabatt.

### B 1: Geschützter digitaler Bereich vor der Konsultation - nach Einrichtung eines persönlichen Benutzerkontos und entsprechendem Identity Management sowie Datenschutz zugänglich

**6. Handhabung bzw. Managen der Patienten während der akuten, adaptiven und digitalen Beschwerdeanamnese:** Dieses Modul als Patienteninteraktionsmodul bzw. als eine Teilkomponente davon kann den Identitäts-Managementprozess, das Einverständnis sowie Datenschutzerklärungen, vor allem aber einen adaptiven Fragebogen, der die aktuellen Beschwerden und die Dringlichkeit bzw. das Gesundheitsanliegen erfasst, umfassen. Der adaptive Fragenbogen kann so konstruiert sein, dass er unter Nutzung mobiler Endgeräte problemlos bearbeitet werden kann, nur wenige Minuten Zeit beansprucht und durch eine einem Entscheidungsbaum ähnliche Struktur, mit Möglichkeit zur Adaptation nach jeder einzelnen Frage rasch zu einer Triage und Einschätzung der Dringlichkeit führt. Dabei können die Anwendungen entweder browserbasiert und damit in Abhängigkeit von einer Internetverbindung oder auch direkt in der Anwendung auf dem mobilen Endgerät angeboten werden.

**7. Stratifizierung und Triagierung von Patienten für die akute oder vereinbarte Behandlung im Gesundheitszentrum/-praxis (Expertensystem/künstliche Intelligenz als digitales Medizinprodukt zur Stratifizierung und Triage nach Dringlichkeit):** Dieses Modul als Priorisierungsmodul des Patientenmanagementsystems dient dazu, akute und dringliche Behandlungsanlässe schnell einer telemedizinischen Konsultation zuzuführen und die bereits erhaltenen Informationen einem real existierenden telemedizinischen Arzt (vgl. Punkt 10.) strukturiert und verständlich darzubieten. Sofern aufgrund des als digitales Medizinprodukt zuzulassenden Systems keine Dringlichkeit der unmittelbaren Behandlung besteht, erfolgt die Weiterleitung auf den ausführlichen Basisfragebogen und den Ressourcen-Fragebogen, die Eingangsvoraussetzung sein kann, um in der Arztpraxis überhaupt ein Termin erhalten zu können. Auf diese Weise kann sichergestellt werden, dass zukünftige Patienten in aller Ruhe in ihrem häuslichen Umfeld die notwendigen Informationen für eine vertiefte Beratung und Begleitung in Gesundheitsförderung, Prävention und Behandlung verfügbar machen und diese vor der Konsultation ausgewertet werden kann und dass andererseits keine akute Problematik, welche einer dringlichen Behandlung bedarf verpasst wird.

**8. Adaptive Erfassung von Ressourcen, Präferenzen, Persönlichkeit und gesundheitsstärkenden Faktoren zur Individualisierung der Empfehlungen:** Es kann ein gezieltes Erfassen der Ressourcen der zukünftigen Patienten, ihrer Präferenzen, und sofern eine entsprechende Zustimmung vorliegt auch der Persönlichkeit mittels wissenschaftlich validierter Instrumente erfolgen. Dies ist bedeutsam, da empirische Daten vorliegen, dass die Berücksichtigung individueller Persönlichkeitsmerkmale etwa bei Vorschlägen zur Verhaltensveränderung aber auch in der Behandlungsführung selbst zu deutlich verbesserter Compliance und verbesserten Outcomes führen sowie geringerer zeitlicher Inanspruchnahme der beratenden Personen. Außerdem kann dies die Eingangstür zu digitalen Angeboten zur Stärkung von Ressourcen darstellen. Da gerade in der hausärztlichen Versorgung unklare Beschwerdebilder ohne fassbare medizinische Ursache ausgesprochen häufig vorkommen, bieten sich hier bereits im Vorfeld Ansatzpunkte für die gesundheitsstärkende und die Gesundheitskompetenz verbessernde digitale Lösungen, welche ihrerseits wieder die zeitliche Inanspruchnahme des Personals in der Praxis reduzieren.

**9. Adaptiver Basisfragebogen zur Gesundheit, zu Belastungen und medizinischer Vorgeschichte:** Der Basisfragebogen kann eine ausführliche Erhebung der Vorgeschichte, der Beschwerden, der aktuellen psychischen Situation, des psychosozialen Umfeldes, der Arbeitssituation (in einer Weise, wie sie eigentlich bei einer Erstanamnese von neuen Patienten in einer hausärztlichen Praxis stattfinden sollte, aber aufgrund der aktuellen Vergütungsstrukturen in dieser Tiefe praktisch niemals stattfindet) umfassen. Dabei können computeradaptive Fragebögen eingesetzt werden, welche die ständige Interaktion mit einem entsprechenden Algorithmus / Expertensystem / Künstlicher Intelligenz (KI) erfordern. Durch diese vorteilhafte Ausgestaltung, bei welcher unter anderem Sachkenntnis zur Optimierung von Fragebögen mittels Item-Response-Theorie und Bayes-Entscheidungs-Algorithmen zum Einsatz kommen kann, wird die Ausfallzeit für die Betroffenen gegenüber dem einfachen Einsatz der Originalfragebögen auf ein Drittel reduziert. Dies steigert die Benutzerzufriedenheit und die Adhärenz.

**10. Telemedizinische Akutkonsultation:** Sofern die unter Punkt 7 oder 11 eingesetzten automatisierten Module bzw. Expertensystem-Module eine hohe Wahrscheinlichkeit für eine akute Behandlungsdringlichkeit finden, wird die Person weitergeleitet für die Kontaktaufnahme einer telemedizinischen ärztlichen Konsultation durch entsprechende Expertinnen und Experten aus dem Praxisnetzwerk. Diese telemedizinische Konsultation kann unter Zugriff auf alle für die betroffene Person vorhandenen Daten erfolgen, mit entsprechender Visualisierung auf dem Bildschirm der ärztlichen Beratungsperson. Diese ärztliche Beratungsperson ist aus organisationsnahen Gründen nicht zwingend identisch mit dem in der Arztpraxis arbeitenden ärztlichen Team, sondern kann in einer vorteilhaften Ausführungsform einen übergeordneten notärztlichen Dienst darstellen, der mehrere derartige Gesundheitspraxen versorgt. Dies beinhaltet auch die Möglichkeit, telemedizinisch ein Rezept auszustellen und dies gegebenenfalls bei einer Versandapotheke einzulösen. Ebenso können, sofern sachlich gerechtfertigt, bestimmte Bescheinigungen etwa über Arbeitsunfähigkeit auf diese Weise ausgestellt werden.

**11. Erstellung einer ganzheitlichen Gesundheitsbeschreibung der Benutzer/Customer/Patienten (Expertensystem/künstliche Intelligenz zur Auswertung der Daten gewonnen unter den Punkten 6, 8 und 9):** Das hier eingesetzte Modul/Expertensystem in Form eines Auswertungsmoduls braucht nicht zwingend als digitales Medizinprodukt zugelassen zu sein, da es zunächst nur Informationen liefert und keinen Eingriff in Behandlungsentscheide vollzieht. Das Auswertungsmodul kann in einer vorteilhaften Ausgestaltung auch als digitales Medizinprodukt zugelassen werden und könnte dann die Behandlungsführung direkt unterstützen. Davon hängt unter anderem die Steuerung des Algorithmus für die Auswahl der zu stellenden Fragen ab.

**12. Visualisierungsmodul zur Erstellung einer persönlichen individualisierten Auswertung aus Punkt 11:** Ein wesentliches Ergebnis des Visualisierungsmoduls kann die Erstellung eines individuellen Informationsberichtes einschließlich verständlicher Visualisierungen, in einer vorteilhaften Ausgestaltung beispielsweise als Radar Chart, sein. Das Visualisierungsmodul bezieht dabei seine Informationen aus dem Modul gemäß Punkt 11 und müsste aufgrund der reinen Information nicht zwingend als digitales Medizinprodukt zugelassen sein. Dabei können sowohl die Ressourcen als auch die Belastungen dargestellt werden. In den Informationstexten können Hinweise für die Nutzung von den unter Punkt 14 beschriebenen digitalen Gesundheitscoachs gegeben.

**13. (12a: KI) Modul zum Matching von Patientenpräferenzen, Raumbedarf, Arztmerkmalen für die Ermittlung des Zeitbedarfs für Konsultation mit Terminvergabe (Terminbuchungs- und Matching-System):** Die in 6, 8 und 9 erhobenen Daten mit Verarbeitung in 11 können in ein Terminbuchungssystem eingehen, welches einerseits eine Prognose über die mutmaßliche Dauer des erforderlichen Beratungsgespräche erstellt und damit eine adaptive Terminbuchung ermöglicht und andererseits auch aufgrund der Ziele des ärztlichen Personals einen Best-Match versucht zwischen der ärztlichen Expertise und dem Beratungs- oder Behandlungsanliegen. Dies könnte auch zur Folge haben, dass gar kein ärztlicher Termin vereinbart wird, sondern beispielsweise ein Termin mit einer Physiotherapeutin, einer Psychologin oder einer in der Gesundheitspraxis arbeitenden Ernährungsberaterin. Bei entsprechender Strukturierung in der Gesundheitspraxis kann die Leistung dennoch als in Delegation erbracht abgerechnet werden. Dieses Modul könnte auch mögliche Nachkonsultationen steuern bzw. planen und kann für das ärztliche und nicht-ärztliche Personal realistische Zeitvorgaben machen. Dieses Modul kann daher auch das personale Ressourcen-Management der Gesundheitspraxis enthalten und im Kern der Prozess-Optimierung stehen. Dieses System kann auch den Vergleich von Soll und Ist sowie Key Performance Indikatoren und Schlüsseldaten für das Process-Mining liefern.

**14. Erste Angebote digitalen Gesundheitscoachings aufgrund der Ergebnisse von 11:** Viele Anliegen, insbesondere gerade zur Ressourcenstärkung aber auch bei banalen Behandlungsanliegen, wie etwa Erkältungskrankheiten profitieren von Informationen, die in Form eines digitalen Gesundheits-Coaches dargeboten werden kann und auf diese Weise Beratungszeit des nichtärztlichen oder ärztlichen Personals in der Gesundheitspraxis einspart. Verknüpft mit der Möglichkeit der telemedizinischen Konsultation entsteht so eine Customer Journey, bei welcher die Patienten vollständig im digitalen Raum den gesamten Behandlungszyklus durchlaufen können und erst bei ausbleibender Besserung die Praxis aufsuchen müssen.

### C: Der analoge Bereich der realen ärztlichen Praxis.

**15. Empfang und Check-in in der Praxis aufgrund von Terminvergabe unter 13 oder 10:** In einem vorteilhaften Ausführungsbeispiel der Erfindung ist eine ganz auf die hybriden Prozesse ausgerichtete Innenarchitektur der Arztpraxis vorgesehen. In dieser Innenarchitektur werden vorhandene Erkenntnisse über gesundheitsförderliche Formen, Materialien, Licht eingebracht. Zudem wird dem Umstand Rechnung getragen, dass Patienten immer stärker eine Erwartungshaltung haben als Kunde auf Augenhöhe behandelt zu werden und nicht als hilfesuchender Bittsteller. Dieser bereits vorher ausgeführten Veränderung in der Einstellung der Menschen insgesamt zu Gesundheit und Gesundheitsversorgung muss durch eine innenarchitektonische Struktur im Empfangsbereich Rechnung getragen werden, sodass allein schon das Ankommen in der Praxis eine positive emotionale Stimmung induziert. In vorteilhafter Weise können Elemente der Räumlichkeit mit einer entsprechenden persönlichen individualisierten Begrüßung am Empfang bereits zu einer Entspannung beim Gast, des Patienten oder dem Hilfesuchenden führen, die im weiteren Verlauf des Aufenthaltes in der Arztpraxis zu einer Einsparung an Gesprächszeit beim besonders teuren ärztlichen Personal führen können und auf diese Weise in der gesamtbetriebswirtschaftlichen Betrachtung das höhere Investment für die Einrichtung und die großzügigere Räumlichkeit rechtfertigen.

**16. Arrival Lounge und Genius-Desk für die Abklärung von Fragen zur elektronischen Patientenakte oder Fragen zur Gesundheitskompetenz:** Ein besonderes Merkmal der Arztpraxis im Rahmen eines Verfahrens zum Patientenmanagement gemäß einem Ausführungsbeispiel der Erfindung ist, dass grundsätzlich keine Warteräume vorgesehen sind. Allein schon aufgrund der durch Covid-19 bestehenden erhöhten Ansteckungsgefahr wäre es obsolet, gesunde Besucher und Patienten dieser Arztpraxis in einem Wartebereich auf engen Raum zu mixen. Daher ist grundsätzlich vorgesehen, dass jede eintreffende Person nach der Begrüßung in ihren persönlichen Gesprächsraum begleitet wird, der unter Punkt 18 detailliert beschrieben ist. Sofern überhaupt Wartezeiten erforderlich sind, finden diese im persönlichen Gesprächsraum statt. Gleichwohl ist vorgesehen, einen angenehmen Bereich zu schaffen gleich einer Arrival Lounge in erstklassigen Hotels, in welchem in Analogie zu Apples Genius Bar die Möglichkeit besteht, Personen die in der Anwendung der digitalen Lösungen noch nicht kompetent sind oder dort Hilfebedarf haben, zu instruieren und zu begleiten. Insbesondere ist vorgesehen, hier den zu erwartenden erschwerten Verständnis-Zugang zur elektronischen Patientenakte zu erleichtern und zu vermitteln. Die Arrival Lounge kann außerhalb der regelhaften Betriebszeiten der Arztpraxis auch für Gruppenschulungen eingesetzt werden und kann mit entsprechenden digitalen Visualisierungsunterstützungen eingerichtet sein. Da im Rahmen des Ausführungsbeispiels bewusst auf ein Wartezimmer verzichtet wird, verkörpert die Arrival Lounge zusätzlich den für einige Patienten wichtigen Sozialraum zum persönlichen und informellen Austausch.

**17. Optimierung adaptiver Raumelemente und baulicher Strukturen auf Basis von Raumsignalen, Präferenzen der Anwender, Patientencharakteristika und/oder digitalen Biomarkern (Expertensystem/künstliche Intelligenz zur individuellen Abstimmung adaptiver Raumelemente wie beispielsweise Licht und Hintergrundklang auf den Patienten, Visualisierung der relevanten Patienteninformationen für den Patient im Gesprächszimmer, Personenleitsystem und Tracking von Patientenparametern mittels digitaler Biomarker, z.B. Stimme):** Die Praxisabläufe und die Interaktion mit einem cloud-basierten Patienteninformationssystem als Teil eines Patientenmanagementsystems gemäß einem Ausführungsbeispiel der Erfindung können durch eine eigene Instanz einer künstlichen Intelligenz bzw. eines Expertensystems-Modul gesteuert werden. Dieses Systemmodul kann dafür zuständig sein, im jeweiligen Gesprächsthema die individuellen Daten der Betroffenen/Patienten sowie die Visualisierung aus Punkt 11 und 12 darzubieten und beispielsweise auf ausliegenden Tabletts QR-Codes anzuzeigen, welche zu vertiefenden Informationen zur spezifischen Situation des Betroffenen bzw. Patienten leiten. Diese können mit mobilen Endgeräten während der Wartezeit aufgerufen werden. In dieses Expertensystem-Modul können die nichtärztlichen und ärztlichen Teammitglieder ihre Informationen und Befunde eingeben, welche dann über FHIR (Fast Healthcare Interoperability Resources) kompatible Schnittstellen in ein Praxisinformationssystem, in Systeme zur Patienten-Nachverfolgung, in ein Prozess-Mining und/oder in Abrechnungssysteme eingehen können. Im Rahmen einer vorteilhaften Ausgestaltung kann dieses Modul als Raumanpassungsmodul die Lichtfarbe und Beleuchtung des Gesprächszimmers, eine Hintergrundmusik und/oder Hintergrundklänge steuern, um auf diese Weise eine individualisierte, auf die spezifische Situation der Betroffenen angepasste Raumsituation zu schaffen. Dazu kann auch der Einsatz von digitalen Biomarkern unter Nutzung einer Aufzeichnungsfunktion etwa mobiler Endgeräte, z.B. zur Analyse der Stimme, gehören.

**18. Gesprächszimmer mit adaptiven Raumelementen (z.B. Akustik, Licht, Klang, und/oder Duft):** Da in der Regel die Hauptzeit der ärztlichen Interaktion für das Gespräch aufgewendet wird und nicht für die klinische Untersuchung, können in der Arztpraxis mehrere reine Gesprächszimmer vorgesehen sein. Diese können einem Raumkonzept bezüglich Sitzposition der Betroffenen und der ärztlichen oder nichtärztlichen Mitarbeitenden Rechnung tragen, wie es aus der psychologischen Forschung zur Verhaltensänderung zu Familiensystemen als vorteilhaft bekannt ist. Da ein reines Gesprächszimmer deutlich geringeren Raumbedarf hat, als ein - nach derzeitigem Stand der Technik übliches - Arztzimmer mit Untersuchungsliege und etwa Ultraschallgerät, wird durch diese Ausführungsform der Erfindung die Raumnutzung optimiert. So kann statt eines Wartebereiches jeder Besucher der Arztpraxis vom Check-in gleich in das durch Licht, Klang und Visualisierungen personalisierte und individualisierte Gesprächsumfeld geleitet werden. Optional ist denkbar, spezifische Düfte einzusetzen, da wissenschaftlich die besondere Wirksamkeit gerade von sublimal wahrgenommenen Geruchsempfindungen bekannt ist. Dies alles kann dazu dienen, den sogenannten Placeboeffekt, dem in der hausärztlichen Betreuung oft ein wesentlicher Anteil am Behandlungserfolg zukommt, systematisch zu verstärken.

**19. Separates Untersuchungszimmer:** Im Rahmen eines Ausführungsbeispiels der Erfindung ist ein separates Untersuchungszimmer vorgesehen, in welchem alle Vorgänge stattfinden, bei denen eine Interaktion mit dem Körper der Betroffenen stattfindet, angefangen von Blutentnahme über Ultraschalluntersuchung bis zu klinischer Untersuchung. In einer weiteren vorteilhaften Ausgestaltung der Erfindung ist denkbar, hier von vornherein eine Unterscheidung nach Untersuchungsräumen für Männer und für Frauen zu treffen und auf diese Weise eine für die oft schambehaftete Untersuchungssituation vorteilhaftere Atmosphäre zu schaffen und damit das gesamte Benutzererlebnis des Praxisbesuches zu verbessern.

**20. Matching von sozialen und Outcome Daten mit den klassischen Daten der Patienteninformationssysteme und/oder Praxissysteme:** In einer vorteilhaften Ausgestaltung der Erfindung kann das System zur Integration etwa auch von telemedizinischer Konsultation mit Spezialisten getrennt von dem in Punkt 17 beschriebenen Expertensystem vorgesehen sein, um über FHIR-kompatible Schnittstellen einerseits die Integration in das Praxisinformationssystem zu gewährleisten und gleichzeitig auch die Ablage von neuen Informationen in der persönlichen elektronischen Krankenakte der Patienten zu ermöglichen.

**21. Gesprächszimmer wie unter Punkt 18 beschrieben für ein zweites Gespräch zur Integration von Befunden oder telemedizinische Konsultation mit einem externen, hinzugezogenen Experten:** Dieses Gesprächszimmer kann identisch zu dem unter Punkt 18 beschriebenen Gesprächszimmer ausgeführt sein und ist aus Gründen der Prozessdarstellung separat aufgeführt. In einer vorteilhaften Ausgestaltung der Erfindung kann vorgesehen sein, dass nach einer Erhebung von Befunden durch nichtärztliches Fachpersonal gegebenenfalls auch telemedizinisches Hinzuziehen von fachärztlichem Personal als ein zweiter Abschnitt der Konsultation stattfindet, in welchem der vorläufige Behandlungsplan festgelegt wird und den Betroffenen die vorläufige Arbeitshypothese (Diagnose) eröffnet und erklärt werden kann. Dabei kann bereits hier auf die ausführlichen im digitalen Raum vorhandenen und personalisierbaren Informationen sowie Erklärungsvideos verwiesen werden, da bekannt ist, dass Patienten insbesondere bei schweren Erkrankungen nur minimale Inhalte des ärztlichen Aufklärungsgesprächs langfristig memorisieren können. Somit kann ermöglicht werden, dass die Bewältigungskompetenz der Betroffenen nach der Konsultation deutlich höher ist als in bisherigen hausärztlichen Versorgungssystemen nach dem Stand der Technik.

**22. Patientenlenkung für das systematische Nachverfolgen der Patienten auf Basis der Differentialdiagnose und Behandlungshypothese (Expertensystem für Planung des Follow-Up und Behandlungshypothese):** Aus Gründen der Darstellung ist dieses Expertensystem-Modul für die Planung einer Nachsorge und Nachverfolgung separat dargestellt zu den Expertensystemen-Modulen unter Punkt 17 und 20, welche eine leicht unterschiedliche Funktionalität bedienen. In einer vorteilhaften Ausgestaltung der Erfindung, insbesondere die digitalen Lösungen betreffend, kann dieses Expertensystem-Modul als Mikroservice auf entsprechend konfigurierten Servern im Backend laufen und als möglicherweise eigenständiges und ggf. als Medizinprodukt zertifiziertes System zur Behandlungsnachsorge beitragen. Insbesondere eine erforderliche Nachbefragung der Betroffenen induzieren und mögliche digitale Lösungen der Organisation und Versorgung mit Medikamenten und anderen Hilfsmitteln steuern.

**23. Check-Out:** Im Rahmen eines Ausführungsbeispiels der Erfindung kann jede betroffene Person am Check-Out eine ausgedruckte schriftliche Zusammenfassung erhalten, welche von dem Expertensystem-Modul in Punkt 21 generiert wird und entsprechende QR-Codes als Zugang zu Informationen im persönlichen digitalen Raum (d.h. im Rahmen des Benutzerkontos des Patienten) sowie dem öffentlichen zugänglichen System der Arztpraxis enthält. Damit kann dem Umstand Rechnung getragen werden, dass nur wenige Patienten in der Lage sind, die Informationsfülle aus einem Arztbesuch adäquat ins Langzeitgedächtnis zu überführen und daraus Handlungen abzuleiten. Dies gilt insbesondere dann, wenn vielschichtige Themen einschließlich dem Stärken von Ressourcen adressiert werden.

### B 2: Geschützter digitaler Bereich nach der Konsultation - erst nach Einrichtung eines persönlichen Benutzerkontos und entsprechendem Identity Management sowie Datenschutz zugänglich

**24. Systematischer Follow-up nach Behandlungsanlass und Hypothese mit digitalem und ggf. persönlichem telemedizinischem Follow-up:** Ein wesentlicher Unterschied einer vorteilhaften Ausgestaltung der Erfindung zu bisherigen Praxen und Verfahren gemäß dem Stand der Technik ist, dass jeder Besucher der Arztpraxis in Abhängigkeit vom Behandlungsanlass zur regelmäßigen Nachbefragung aufgefordert wird. Dies kann beispielsweise bei akuten Infektionskrankheiten zweimal täglich sein, bei chronischen Verläufen in größeren Abständen, in jedem Fall so strukturiert, dass computergestützte Expertensysteme-Module daraus ableiten können, mit welcher Wahrscheinlichkeit der beobachtete Verlauf mit dem unter der Annahme der richtigen Diagnose und richtigen Arbeitshypothese zum erwarteten Verlauf passt oder ob eine alternative Diagnose zutreffender ist. Ferner wird hier ersichtlich, welche weitere Unterstützung aus den vorhandenen Möglichkeiten für eine erfolgreiche Verhaltensänderung die beste Verstärkung bietet. Dieses Vorgehen kann ergänzt werden durch telemedizinische Nachkonsultation bei entsprechender Indikation.

**25. Regelmäßige situationsgerechte Nachbefragung des Patienten über Verlauf und Veränderung als Basis für ein Process-Mining:** Die unter Punkt 24 beschriebene systematische Nachbefragung kann die Basis für ein Process-Mining entlang der gesamten gesundheitsbezogenen Wertschöpfungskette von Wohlbefinden über Prävention und Frühdiagnose bis zu Behandlung und Nachsorge bilden. Hier unterscheidet sich die Nachsorge gemäß einem Ausführungsbeispiel der Erfindung wesentlich von der bislang geübten Praxis nach dem Stand der Technik und erweitert die aus der Praxis bekannte Lehre bedeutsam um die erstmalige Möglichkeit, individualisiert und auf den jeweiligen Kontext der betroffenen Personen bezogen, durch künstliche Intelligenz optimale Behandlungspfade zu entwickeln.

**26. Begleitung von Patienten durch ihren gesunden Lebensalltag in Form eines Gesundheitscoachs/digitale Gesundheitsanwendungen (ggf. rezeptpflichtig) für Veränderung und Begleitung:** Ein wesentlicher Bestandteil der Nachsorge gemäß einem Ausführungsbeispiel der Erfindung sind digitale Gesundheitsanwendungen, die von der Gesundheitspraxis verordnet werden und durch das unter Punkt 24 und 25 beschriebene System auch einer systematischen Erfolgskontrolle zugänglich werden. Ergänzend können bei Prävention und Gesundheitsförderung digitale Präventionsanwendungen zum Einsatz kommen, die im Grundsatz ähnlich konzipiert sind wie digitale Gesundheitsanwendungen, nur unter anderen Vergütungsstrukturen betrieben werden. Allen diesen Gesundheitsanwendungen können wissenschaftliche Konzepte aus der Verhaltensökonomie und Verhaltensforschung zugrunde liegen.

**27. Telemedizinischer persönlicher Follow-Up bei Bedarf:** Ergänzend zu den rein digitalen Gesundheitsanwendungen können hybride Systeme zum Einsatz kommen, bei denen die persönliche telemedizinische Nachsorge rein digitalen Formate ergänzt. Diese persönliche telemedizinische Nachsorge kann von ärztlichem Personal oder auch von nichtärztlichen Fachpersonen ausgeführt werden. Wesentlich ist, dass diese persönliche telemedizinische Nachsorge zur Stärkung der Beziehung zu Patienten und Besuchern beiträgt und durch die in Punkt 24 bis 26 beschriebenen Vorgehensweisen erlaubt, systematisch eine optimale Frequenz des persönlichen Kontaktes zu ermitteln.

**28. Echtzeitsteuerung der Nachbetreuung für Patienten und Hausärzte/Health Professionals (Expertensystem bzw. KI-Modul zur Steuerung der Nachbetreuung):** Die Nachsorge wird idealerweise, um die Komplexität der Systeme zu reduzieren, von einem eigenen, für diesen Zweck entwickelten Expertensystem-Modul, das Ansätze aus der Applikation von Bayes-Theorem verknüpft mit künstlicher Intelligenz gesteuert. Es kann jeweils neue Information aufgrund der Nachbefragung im Sinne eines Updates der nach Testwahrscheinlichkeit für ein Verhalten oder die Passung eines Verlaufes zum erwarteten Verlauf jeweils erneut, einer Mustererkennung durch künstliche Intelligenz zugeführt werden. Dies erweitert die aktuell nicht dynamisch die Wahrscheinlichkeit updatenden Systeme nach dem Stand der Technik und bedarf der Implementation in leistungsfähigen Rechenzentren.

**29. Präferenzbasierte, Patienten-individuelle Empfehlung von Dienstleistungen und Produkten aus dem Gesundheits- und allgemeinen Lebensbereich, wie z.B. Lebensmittel-Empfehlungen bei Unverträglichkeiten oder des Mikrobioms oder die Anwendung von mittel-frequenter Elektrostimulation bei Beschwerden im oberen Rückenbereich:** Im Rahmen einer vorteilhaften Ausgestaltung der Erfindung kann der Einbezug von Wertschöpfungssystemen aus dem nicht medizinischen Bereich vorgesehen sein. Menschen gehen häufiger Einkaufen als zum Arzt. Die rechtlichen Voraussetzungen erfordern jedoch hier unter Einhaltung von Datenschutzbestimmungen und der persönlichen Selbstbestimmung der Betroffenen digitale Lösungen zu implementieren, welche beispielsweise ein erweitertes Einkaufserlebnis in realen Lebensmittelgeschäften ermöglichen oder aber spezifische, evidenzgesicherte Empfehlungen für digitale Einkaufserlebnisse oder Dienstleistungen ermöglichen. Gerade durch den Fokus auf Gesundheitsförderung und Wellness als Beginn der Customer Journey ist diese sektorübergreifende Ergänzung der Begleitung sinnvoll und möglicherweise von vielen Benutzern des Systems sogar ausdrücklich erwünscht.

**30. Partnerprogramm für gesundheitsförderliche Dienstleistungen und Produkte außerhalb des Gesundheitssektors, z.B. Einkauf von Nahrungsmitteln:** Das Expertensystem-Modul aus Punkt 29 eröffnet die Möglichkeit über Partnerprogramme oder andere Assoziationen reale Wertschöpfungsketten zu eröffnen, an denen sowohl die Besucherinnen und Besucher der Gesundheitspraxis als auch die Gesamtorganisation nutzenstiftend partizipieren können.

In der hausärtzlichen Versorgung ist die persönliche Beziehung besonders bedeutsam.

Zwar bringt das beanspruchte Patientenmanagement-System schon eine starke Verbesserung, Effizienzsteigerung und erhöht die Patientensicherheit. Der weiter beanspruchte Virtuelle Physician Assistent ergänzt und erweitert das System aber um die simulierte persönliche Beziehung.

Der Virtuelle Physician Assistant löst das Problem, wonach der Arzt nicht die Zeit hat, dem Patienten in der wünschenswerten Ausführlichkeit Wissen und Erkenntnisse zu vermitteln, mit dem Patienten in der wünschenswerten Weise in den Austausch zu treten und mit dem Patienten gemeinsam über die bestmögliche Lösung nachzudenken. Hier übernimmt der virtuelle Assistent in einer digital unterstützten Weise die dargelegten Elemente der persönlichen Arzt-Patient-Beziehung und verbessert so neben der Sicherheit und Vermittlung auch die so wichtige Arzt-Patient-Beziehung. Es wirkt durch die Gestaltung tatsächlich so wie im Film der Hilfs-Sherrif der Assistent des richtigen Sherrifs ist.

Der Virtuelle Physician Assistant (VPA) unterstützt Ärtz:innen und Patientinnen darin, rascher zur richtigen Diagnose und Therapie zu finden. Der Kerngedanke ist, mittels virtuellem Assistenten Ärtz:innen und Patientinnen in der Kommunikation, Erläuterung, Motivation, Entscheidungsfindung und beidseitigen Effizienz zu unterstützen.

Dazu nutzt der VPA als hardwareseitige Voraussetzung das Smarthphone mit den darin vorgehaltenen Sensoren, Tablet oder Computer der Betroffenen, die digitalen Werkzeuge der Arztpraxis (Tablet, Computer) sowie Back-End-Server-Strukturen auf welchen die nachstehend beschriebenen Lösungen implementiert sind.

Der Kern der Lösung, der über die grundsätzlich beanspruchte Lehre weit hinausgeht, ist die intelligente Visualisierung der Information, die für Patientinnen und Ärzt:innen die Komplexität der gesamten aus Sensorik und anderen Quellen gewonnen Daten so verdichtet, dass eine raschere Entscheidung für die bestmögliche Therapie und wahrscheinlichste Diagnose ermöglicht wird. Dies beruht auf der Kombination von adaptiven Bayes-Theorem-Ansätzen und auf multivariaten logistischen Regressionsverfahren aufbauenden Prädiktionsalgorithmen. Diese berücksichtigen, dass ein und dasselbe identische Symptommuster in Abhängigkeit von Kontext (z. B. Alter), Umgebung und Umwelt (z. B. der aktuell gerade vorherrschenden Virus-Variante bei Atemwegserkrankungen) ganz unterschiedliche individuelle Vorhersagewerte haben kann. Eine mögliche Ausprägung des Verfahrens ist nachstehend erörtert.

Das Verfahren wird dadurch ergänzt und erhöht die Effizienz der Arzt-Patient-Beziehung, dass alle erforderlichen zusätzlichen Informationen, Erläuterungen und Nachbefragungen digital erfolgen, wie beispielhaft nachstehend ausgeführt. In einem Anwendungsbeispiel stellt der VPA vor der Konsultation adaptiv Fragen an die Betroffenen oder Hilfesuchenden (in deren Muttersprache) und bereitet die Information in der Vorbereitung der Konsultation für die Arztpraxis auf. Der VPA schätzt aufgrund der zusätzlichen Fragen auch die Komplexität des Anliegens ein und erlaubt der Arztpraxis so eine differenzierte Zeitplanung. Die Ärztin oder der Arzt interagiert mit dem VPA, indem sie die mutmaßliche Hauptdiagnose, Behandlung und wichtigste zu unterscheidende oder zu befürchtende Differenzialdiagnose angibt und die gewünschten bzw. mit den Betroffenen in der Konsultation besprochenen weiteren Maßnahmen einschließlich Gesundheitsförderung und allgemeiner Prävention. Der VPA stellt für die Betroffenen einerseits motivierende Erklärvideos in der Muttersprache zur Verfügung und andererseits gezielt Nachbefragung, u.a. zum Verlauf, zur Besserung und zur Unterscheidung zu anderen Differentialdiagnosen.

Der VPA kann die weitere Behandlung in möglichen Ausprägungen wie folgt unterstützen: Im Anwendungsfall "Ernährungsberatung und Gewichtsveränderung" könnte der VPA aufgrund von Geolocation des Smartphones den Aufenthaltsort bestimmen und situationsgerechte Hinweise (z. B. Einkauf von Lebensmitteln geben) liefern. Beim Anwendungsfall Prävention von Erschöpfung und Stressbewältigung könnte der VPA auf Wunsch der Betroffenen aus der Sensorik des Smartphones über Stimmfrequenzanalyse, Bewegungsmuster und Lagesensoren situationsadäquate Hinweise zur Verhaltensmodifikation geben. Beim Anwendungsfall "Findung der bestverträglichen und bestwirksamen Medikamentenkombination etwa bei der Einstellung eines Blutdrucks" kann der VPA die Konzepte der vergleichenden N=1 Studien praktisch umsetzen, und Patientinnen und Ärzt:innen durch spezifische Tagebücher mit hinterlegter statistischer Auswirkung bedeutsam effektiver darin unterstützen, die optimale Medikation zu finden. Bei allen Anwendungsfällen ist bedeutsam, dass das System sowohl den Input von Sensorik als auch von Patientinnen und Ärzt:innen sinnvoll integriert. Zu jedem Zeitpunkt kann die Arztpraxis neue "Hinweise" an den VPA übergeben, die wiederum die Fragen, Erklärungen, Mitteilungen des VPA an die Betroffenen beeinflussen. Der VPA entlastet auf diese Weise die Arztpraxis in der Vorbereitung und Nachsorge von Konsultationen und steigert so die Wirksamkeits-Effizienz der Konsultation. In einer Ausbaustufe werden die Bild- und Sprachwelten nicht nur kultursensitiv, sondern auch persönlichkeitssensitiv, d.h. leicht unterschiedliche Aufbereitung nach fünf Hauptpersönlichkeitstypologien.

Technologisch basiert der VPA auf adaptiver Erfassung von Fragebögen (Patient Reported Outcome Measures, PROMs) sowie Entscheidungsbäumen und Rückmeldung sowohl an die Betroffenen wie auch an die Arztpraxis. Dazu werden standardisierte, Gematik-konforme Schnittstellen zu Praxisinformationssystemen genutzt. Bestimmte Datenkonstellationen verbunden mit Eingaben durch die Arztpraxis generieren regelbasiert Outputs des VPA an die Betroffenen und geben der Arztpraxis u.a. Wahrscheinlichkeiten für Differentialdiagnosen. Diese Regeln werden in der Back-End-Datenbank regelmäßig durch KI-Applikationen überprüft, wobei solange KI-basierte Prozesse nicht MPG zulassungsfähig sind, die KI als Output eine Anpassung der Regelwerke vorschlägt, die von einem Expertengremium geprüft und dann in regelmäßigen Updates in das System eingespielt werden.

Vier Kernelemente des Verfahrens sind der "Radarchart", die "Heatmap", die "kontextspezifische Prädiktion" und das "N=1-Verfahren", die nachstehend beschrieben sind.

Der "Radarchart" baut auf bekannte Spinnendiagramme auf, erweitert diese jedoch durch statistische Verfahren und Darstellung auf Endgeräten bedeutsam. Im Kern ist der Radarchart eine multivariate Darstellung verschiedener für die Gesundheit relevanter Faktoren, die üblicherweise in einer langen ausführlichen und die psychosozialen Rahmenbedingungen ebenfalls berücksichtigenden Anamnese erfragt werden. Hier wird die Anamnese durch Algorithmus gesteuertes adaptives Fragen ähnlich einem guten Arztgespräch erhoben und so visualisiert, dass die rote Referenzlinie den Erwartungswert für gleich alte Personen mit gleichem Geschlecht und Hintergrund aufzeigen und alle Faktoren auf eine einheitliche Metrik standardisiert sind (gleiche Standardabweichung für alle Skalen). Dies erlaubt auf einen Blick den gesamten kontextuellen Hintergrund des Betroffenen und seine gesundheitliche und Beschwerden-Situation zu visualisieren und den Verlauf durch Einfügen zusätzlicher Linien darzustellen. Der "Radarchart" eignet sich komplexe, aber über kürzere Zeiträume stabile Verhältnisse im Querschnitt darzustellen.

Bei Infektionskrankheiten oder anderen akuten Behandlungsanlässen jedoch ändert sich die Symptomatik rasch, dafür ist der "Radarchart" weniger geeignet. Hier kommt die "Heatmap" zum Einsatz, die beispielsweise Tag für Tag die Intensität der wichtigsten bei einer Erkrankung möglichen Symptome oder Biomarker auf einer mehrstufigen Skala erfasst und den Verlauf anschaulich visualisiert. Technisch ergibt sich eine n x m Matrix aus Datenpunkten, welche ein Muster zeigt, das sich in der Zeit entwickelt. Dieses Muster kann algorithmisch mittels einfacher statistischer Verfahren, z. B. Chi-Square-Analyse mit Referenzmustern unterschiedlicher Erkrankungen verglichen werden.

Mit jedem Beobachtungszeitraum steigt die Information und dabei die Möglichkeit, das beste passende Muster und damit die richtige Differenzialdiagnose frühzeitig zu erkennen. Die von der Arztpraxis als das beste passende Muster = mutmaßliche Diagnose ausgewählte Entität erlaubt nun im weiteren Verlauf eine Vorhersage über die zu erwartenden Ausprägungen von Symptomen und Biomarkern und jeweils den upgedateten Vergleich mit dem tatsächlich beim Betroffenen vorliegenden Geschehen.

Folgt der aktuelle Fall dem Muster der Referenzfälle für diese Diagnose so errechnen die Algorithmen einen geringen Misfit, bei einem abweichenden Verlauf entsteht etwa aus der Chi-Square-Statistik frühzeitig ein hoher Abweichungswert, der die Arztpraxis darauf aufmerksam macht, dass die gewählte Diagnose möglicherweise nicht passt. Hier können subgruppenspezifische Referenzmuster gewählt werden, z. B. das Erwartungsmuster einer Influenza bei einer geschwächten älteren Patient:in im Vergleich zu jüngeren Betroffenen. Der besondere Vorteil der n x m Matrix ist, dass diese Operationalisierung sehr einfach für Methoden des maschinellen Lernens (Künstliche Intelligenz, etwa neuronale Netze) verwendet werden kann. Auf diese Weise entsteht rasch ein gut differenzierendes Set an Referenzdatensätzen, die auch jeweils an veränderte Situationen angepasst werden können, wie etwa die sich verändernde Symptomatik der unterschiedlichen Virus-Mutationen beim SARS-Cov-2 Virus.

Die "kontextspezifische Prädiktion" berücksichtigt, dass das genau gleiche Symptommuster abhängig von anderen Faktoren eine ganz unterschiedliche Aussagekraft haben kann, wie nachstehendes Beispiel erläutert: Ist aktuell in einer Region die vorherrschende respiratorische Infektion durch RSV-Virus ausgelöst und SARS-Cov-2 spielt gerade eine untergeordnete Rolle, so ist es a priori viel wahrscheinlicher, dass eine Person, welche die Arztpraxis wegen neuen respiratorischen Symptomen aufsucht eine RSV-Infektion hat, als eine SARS-Cov-2 Infektion. Kommt die gleiche Person jedoch gerade aus dem Urlaub zurück aus einer Region mit hoher SARS-Cov-2 Prävalenz, so ist eine SARS-Cov-2 Infektion a priori wahrscheinlicher.

Das gleiche Symptommuster bei einer dreißigjährigen Berufstätigen mit Rückenschmerz wird sehr viel wahrscheinlicher durch eine muskuläre Verspannung als der Schmerz bei zuvor blander Anamnese bei einer siebzigjährigen Person. Hier muss auch an eine metastatierendes Malignom gedacht werden. In Kombination mit der "Heatmap" entsteht so sehr viel rascher der Verdacht, dass etwa bei der siebzigjährigen Person keine blande muskuläre Verspannung vorliegt.

Technologisch werden im Hintergrund dazu Server mit hoher Rechenkapazität benötigt, um diese Bayes-Theorem-Algorithmen in Realtime anzuwenden. Für die Übertragung vom mobilen Endgerät zum Back-End-Server ist jedoch nur geringer Informationsfluss erforderlich. Bei weiteren technologischen Fortschritten ist auch denkbar, die Rechenverfahren unmittelbar auf dem Endgerät ablaufen zu lassen.

In vielen Behandlungsfällen, insbesondere bei chronischen Erkrankungen oder Risikokonstellationen, stehen heute vielfältige Medikationen zur Verfügung, die sich in ihrem Wirkungsprofil wie auch Nebenwirkungsprofil individuell stark unterscheiden. Zwar besteht Hoffnung, dass in Zukunft etwa genetische Untersuchungen eine bessere Vorhersage der besten Medikamentenkombination etwa zur Behandlung des Bluthochdrucks möglich sein könnten, dies ist jedoch von der Realität noch weit entfernt. Daher muss in der Praxis - und dazu fehlen Zeit und Instrumente - durch Versuch und Irrtum für jede Patient:in idealerweise individuell die optimale Medikation gefunden werden. Der "N=1 Versuch" unterstützt dies, indem die Arztpraxis auf der Basis der Präferenzen der Betroffenen jeweils zwei Medikamentenkombinationen verschreibt, die nacheinander für eine angemessene Zeit, beim Bluthochdruck beispielsweise je sechs Wochen, verschrieben werden. Die Betroffenen führen auf ihrem Endgerät ein digitales Tagebuch über die Wirkung (hier Blutdruck) sowie die Verträglichkeit (hier Nebenwirkungen). Bereits während der zweiten Sechs-Wochen-Periode führt ein Algorithmus auf dem Back-End-Server täglich eine sogenannte Interim-Analyse durch und ermittelt, ob sich mit einer relevanten Irrtumswahrscheinlichkeit eine der beiden Medikationen in Wirkung und Nebenwirkungsprofil als die bessere im Vergleich herausstellt. Spätestens nach Abschluss der zweiten Periode liegt eine eindeutig interpretierbare Information vor, welches der beiden Vorgehen zu bevorzugen ist.

Wird die Basis-Information aus "Radarchchart" und "Heatmap", kontextspezifische Prädiktion sowie der aktuelle Verlauf im N=1 Versuch berücksichtigt kann in einer zukünftigen Ausprägung des Verfahrens eine künstliche Intelligenz bereits ein Ranking der besten möglichen Medikationsvorschläge der Arztpraxis zum Entscheid unterbreiten.

Hinsichtlich weiterer vorteilhafter Ausgestaltungen des erfindungsgemäßen Patientenmanagementsystems und des Verfahrens zum Patientenmanagement wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

## Patentansprüche

1. Patientenmanagementsystem, insbesondere zur Unterstützung des Arztpraxisbetriebs, vorzugsweise im Bereich der Allgemeinmedizin und/oder der hausärztlichen Versorgung, umfassend:
ein Patienteninteraktionsmodul zur Bereitstellung eines geschützten digitalen Patientenbereichs, wobei das Patienteninteraktionsmodul dazu ausgebildet ist, einem Patienten in Reaktion auf eine Registrierungsanfrage ein persönliches Benutzerkonto zuzuordnen und im Rahmen des persönlichen Benutzerkontos patientenspezifische Daten zu erfassen, und
ein Priorisierungsmodul zur Bestimmung der Dringlichkeit eines Patientenanliegens, wobei das Priorisierungsmodul dazu ausgebildet ist, eine Dringlichkeitsentscheidung auf Basis der erfassten patientenspezifischen Daten zu ermitteln, so dass in Abhängigkeit der Dringlichkeitsentscheidung eine Weiterleitung des Patienten erfolgt.

2. Patientenmanagementsystem nach Anspruch 1, ferner umfassend:
ein Präferenzerfassungsmodul zur Ermittlung von Patientenpräferenzen anhand von gesammelten Nutzerdaten des Patienten, wobei die Nutzerdaten ein Nutzungsverhalten des Patienten hinsichtlich digitaler Dienste und/oder Medien, vorzugsweise aus dem Alltagsbereich, repräsentiert.

3. Patientenmanagementsystem nach Anspruch 2, wobei das Präferenzerfassungsmodul dazu ausgebildet ist, basierend auf den Nutzerdaten des Patienten einen Ist-Zustand und/oder Früh-Indikatoren im Hinblick auf ein geändertes Nutzungsverhalten des Patienten zu ermitteln.

4. Patientenmanagementsystem nach einem der Ansprüche 1 bis 3, ferner umfassend:
ein Telemedizinmodul zur Bereitstellung einer telemedizinischen Konsultation für den Patienten.

5. Patientenmanagementsystem nach einem der Ansprüche 1 bis 4, wobei das Patienteninteraktionsmodul derart ausgebildet ist, dass weitere patientenspezifische Daten erfasst werden, vorzugsweise sofern die Dringlichkeitsentscheidung dies zulässt, und dass die patientenspezifischen Daten in einer Patientendatenbank als Patientenmerkmale hinterlegt werden.

6. Patientenmanagementsystem nach einem der Ansprüche 1 bis 5, wobei die patientenspezifischen Daten Patientenmerkmale hinsichtlich der Ressourcen, der Präferenzen und/oder der Persönlichkeit des Patienten umfassen.

7. Patientenmanagementsystem nach einem der Ansprüche 1 bis 6, wobei die Erfassung der patientenspezifischen Daten mittels einem computeradaptiven Fragebogensystem erfolgt.

8. Patientenmanagementsystem nach einem der Ansprüche 1 bis 7, wobei das Patienteninteraktionsmodul derart ausgebildet ist, dass es mit einem mobilen Endgerät des Patienten interagiert.

9. Patientenmanagementsystem nach einem der Ansprüche 1 bis 8, wobei über eine Sensoreinrichtung des mobilen Endgeräts patientenspezifische Messdaten erfassbar sind, und wobei die patientenspezifischen Messdaten als Patientenmerkmale berücksichtigbar und/oder in einer Patientendatenbank hinterlegbar sind.

10. Patientenmanagementsystem nach einem der Ansprüche 1 bis 9, ferner umfassend:
ein Auswertungsmodul, das dazu ausgebildet ist, basierend auf den patientenspezifischen Daten bzw. den Patientenmerkmalen, eine Zustandsbeschreibung des Patienten zu erzeugen.

11. Patientenmanagementsystem nach Anspruch 10, wobei basierend auf der Zustandsbeschreibung des Patienten ein Matching mit vorgegebenen Merkmalen einer Arztpraxis durchgeführt wird, um eine Abwicklung des Patienten in der Arztpraxis zu steuern.

12. Patientenmanagementsystem nach einem der Ansprüche 1 bis 11, ferner umfassend:
mindestens ein Visualisierungsmodul zur Bereitstellung von Informationen an den Patienten und/oder an einen Arzt bzw. an ein Praxisteam.

13. Patientenmanagementsystem nach einem der Ansprüche 1 bis 12, wobei das Visualisierungsmodul als Nachsorgemodul ausgebildet ist, das - nach einer Arztkonsultation - zur Bereitstellung von Informationen und/oder Anweisungen an den Patienten dient.

14. Verfahren zum Patientenmanagement, insbesondere zur Unterstützung des Arztpraxisbetriebs, vorzugsweise unter Nutzung eines Patientenmanagementsystems nach einem der Ansprüche 1 bis 13,
wobei mit einem Patienteninteraktionsmodul einem Patienten ein geschützter digitaler Patientenbereich bereitgestellt wird,
wobei das Patienteninteraktionsmodul dem Patienten in Reaktion auf eine Registrierungsanfrage ein persönliches Benutzerkonto zuordnet,
wobei durch das Patienteninteraktionsmodul im Rahmen des persönlichen Benutzerkontos patientenspezifische Daten erfasst werden,
wobei mittels einem Priorisierungsmodul die Dringlichkeit eines Patientenanliegens bestimmt wird,
wobei das Priorisierungsmodul eine Dringlichkeitsentscheidung auf Basis der erfassten patientenspezifischen Daten ermittelt, und
wobei in Abhängigkeit der Dringlichkeitsentscheidung eine Weiterleitung des Patienten im Patientenmanagementsystem erfolgt.
